# EUROPEAN PATENT APPLICATION

(11) **EP 3 245 971 A1**
(43) Date of publication of application: **22.11.2017**
(21) Application number: 17171405.8
(22) Date of filing: 16.05.2017
(51) Int. Cl.: A61B 18/12, A61B 18/00

(54) **MULTI-FUNCTION HANDPIECES FOR ENERGY-BASED SURGERY**

(30) Priority: 16.05.2016 US 201662337291 P
(71) Applicant: Omniguide Inc., Lexington, MA 02421 (US)
(72) Inventor: GRAHAM, Marc, Lexington, MA, 02421 (US); ALABRE, Aurore, Lexington, MA, 02421 (US); FUFLYIGIN, Vladimir, Lexington, MA, 02421 (US)
(74) Representative: Intès, Didier Gérard André

(57) **Abstract**

A modular handpiece having a proximal handle module with a handle unit (20) having at least one interior fluid cavity (128), having an exterior gripping surface, defining a fluid control port enabling fluid communication between the atmosphere and the interior fluid cavity (128), and capable of accommodating at least one waveguide for carrying optical radiation and capable of accommodating at least one RF electrode (24, 26). The handpiece further comprises a cannula module (14) having a distal portion (50) with a distal tip (60), a central portion (52), and a proximal portion (54) connectable with the handle module. All three cannula portions (50, 52, 54) are capable of at least one of (a) defining a waveguide conduit, (b) carrying at least one RF electrode, and (c) defining a fluid passageway between the distal tip and the interior fluid cavity (128) of the handle unit.

## Description

### Field

The disclosure relates to surgical instruments that deliver surgical energy, and more particularly to handpieces carrying at least waveguides and/or RF electrodes.

### Description of the Related Art

There are a number of surgical devices utilized by surgeons that deliver one or more types of energy to cut, coagulate, ablate, remove or otherwise treat tissue of a patient. Surgical energy devices include ultrasonic devices, electrosurgical devices utilizing monopolar and/or bipolar RF (RadioFrequency) current, microwave and/or thermal energy devices, and higher-frequency electromagnetic radiation delivery devices such as lasers. Such surgical energy devices are utilized by themselves in some surgical techniques and, in other techniques, are utilized in combination with one or more other tools such as forceps or graspers.

Examples of medical laser systems utilizing hollow waveguides are provided by Temelkuran et al. in U.S. Patent Nos. 7,167,622 and 7,331,954, and by Goell et al. in U.S. Patent No. 8,280,212, all assigned to OmniGuide, Inc. of Lexington, Massachusetts. Utilizing physical spacers or stand-offs when delivering one or more types of surgical energy is disclosed by Farin et al. in U.S. Patent No. 5,776,092 and by Temelkuran et al. in U.S. Patent No. 7,331,954. Other handpieces, including those with counterbalances and waveguide locks, are described by Shurgalin et al. in U.S. Patent Publication No. 2014/0316395A1.

For sterility, safety and quality assurance purposes, each waveguide is utilized for only a single surgical procedure. At the beginning of a procedure, a single-use waveguide typically is inserted into a reusable handpiece which is graspable by a surgeon. Reusable handpieces must be properly sterilized between procedures.

Different types of surgical procedures have differing requirements for length and orientation of instruments utilized during the procedures. Many surgical instruments have been designed to bend in one or more directions to assist insertion and/or to manipulate tissue. Articulating handpieces for waveguides and with additional functionalities are described by Graham et al. in PCT Publication No. WO2016/044640A1.

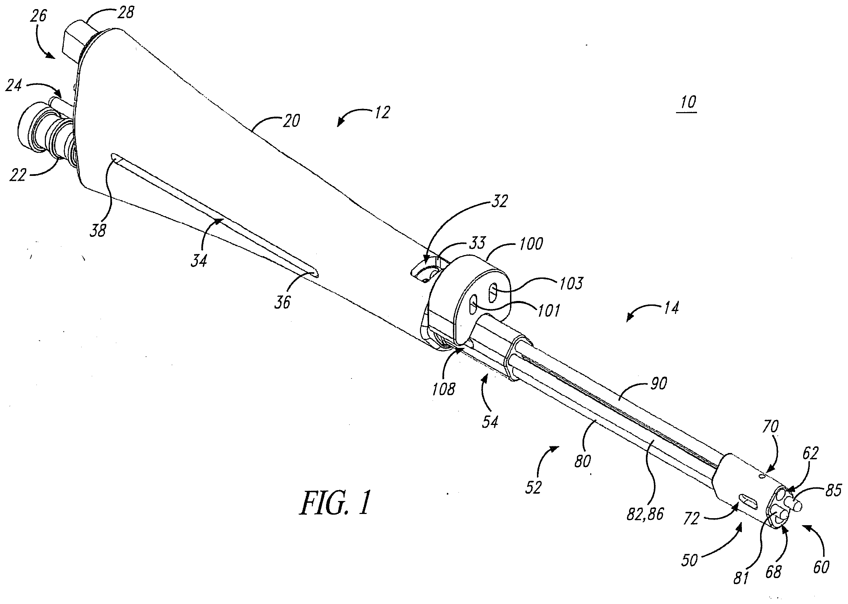

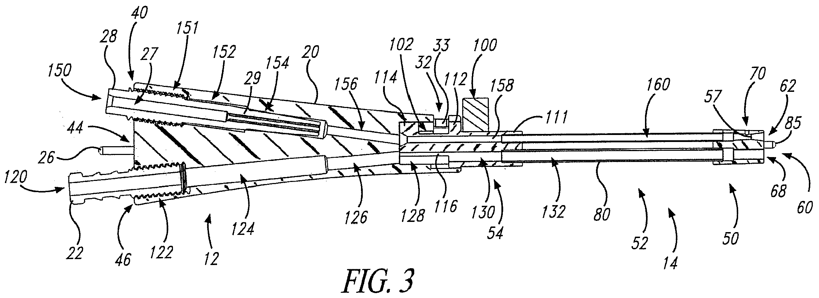

A variety of different distal tips for waveguides within flexible conduits is disclosed by Anastassiou et al. in PCT Publication No. WO2014/043697A2. Several variations of asymmetric distal tips are shown in FIG. 3 of U.S. Patent Publication No. 2014/0088577 by Anastassiou et al. and a standoff tip that is offset to one side of a laser beam axis is shown in FIG. 1 of U.S. Patent Nos. 7,167,622 by Temelkuran et al. Articulated and steerable tips of endoscopic devices and cannulas for robotic surgery are disclosed by Bonneau and by Prisco et al. in U.S. Patent Publication Nos. 2009/0171332 and 2010/0249507, respectively, and by Vargas in U.S. Patent No. 8,075,476.

It is desirable to have improved handpieces to effectively access and treat selected tissue within a patient utilizing surgical energy.

### BRIEF SUMMARY

An improved handpiece for surgical procedures utilizing one or more types of surgical energy is described which is capable of multiple functions including one or more of suction and irrigation. Such a handpiece may readily accept and protect single-use waveguides during surgical procedures. In some implementations, such a handpiece may include one or more of its components designed for single use to avoid cross-contamination from other patients.

This disclosure features a modular handpiece having a proximal handle module with a handle unit having at least one interior fluid cavity, having an exterior gripping surface capable of being grasped by the hand of a user, defining a fluid control port enabling fluid communication between the atmosphere and the interior fluid cavity for fluid management such as suction control, and capable of accommodating at least one waveguide for carrying optical radiation and capable of accommodating at least one RF electrode. The handpiece further comprises a cannula module including a distal cannula portion having a distal tip, a central cannula portion, and a proximal cannula portion connectable with the handle module. All three cannula portions are capable of at least one of (a) defining at least a first conduit alignable with the handle module to carry a waveguide, (b) carrying at least one RF electrode, and (c) defining a fluid passageway between the distal tip and the interior fluid cavity of the handle unit.

In one embodiment, the handle unit is monolithic. In another embodiment, the handle unit is formed from at least two sections or shell pieces that, when assembled together, establish the exterior gripping surface, the interior fluid cavity and the fluid control port. In certain embodiments, the handle unit defines a passage for the waveguide. In some embodiments, the handle module includes at least two RF electrodes, the RF electrodes which are connectable to an RF signal source or generator via a standard RF connector in a number of embodiments. In other embodiments, the handle module includes at least one control switch accessible by a finger of a user and actuatable thereby to control at least one of RF energy delivery and optical radiation delivery.

In one embodiment, the central portion of the cannula module is bendable or flexible. In certain embodiments, the handle module further includes a fitting capable of engaging a conduit leading to a source of negative pressure to create a suction force within the interior fluid cavity when the suction control port is blocked. In some embodiments, at least one portion of the distal tip of the cannula module is at an angle of zero degrees to sixty degrees relative to a reference line perpendicular to a longitudinal axis of the distal cannula portion. In certain embodiments, distal portions of the RF electrodes have a geometry that provides a stand-off distance for laser energy directed through a waveguide, and the distal portions of the RF electrodes preferably enhances the ability to manipulate tissue at the surgical site. In a number of embodiments, the waveguide conduit is mounted above the fluid passageway in at least the cannula module to provide suction below laser energy delivery through the waveguide, when the handle module is gripped and held with a top portion generally facing upward and a bottom portion generally facing downward.

In certain embodiments, one or more portions of the cannula module are shaped and arranged to provide for additional functions, for instance forming forceps, scissors or graspers.

This disclosure also features a handpiece capable of selectively delivering laser energy and RF energy and enabling control of suction, including a handle module with a handle unit having at least one interior fluid cavity, having an exterior gripping surface capable of being grasped by the hand of a user, defining a suction control port enabling fluid communication between the atmosphere and the interior fluid cavity, and capable of accommodating at least one waveguide for carrying optical radiation and carrying at least one RF electrode. The handle module includes a fitting capable of engaging a conduit leading to a source of negative pressure to create a suction force within the interior fluid cavity when the suction control port is blocked. The handpiece further includes a cannula module having a distal portion with a distal tip, a central portion, and a proximal portion connectable with the handle module, wherein all three cannula portions define a waveguide conduit, at least the proximal cannula portion is capable of carrying at least one RF electrode, and all three cannula portions define a fluid passageway between the distal tip and the interior fluid cavity of the handle unit.

This disclosure further features a method of assembling a handpiece, including selecting a handle module to include a handle unit having at least one interior fluid cavity, having an exterior gripping surface capable of being grasped by the hand of a user, defining a fluid control port enabling fluid communication between the atmosphere and the interior fluid cavity, and capable of accommodating at least one waveguide for carrying optical radiation and capable of accommodating at least one RF electrode. The method further includes selecting a cannula module to include a distal cannula portion with a distal tip, a central cannula portion, and a proximal cannula portion connectable with the handle module, wherein all three cannula portions define a waveguide conduit, at least the proximal cannula portion is capable of carrying at least one RF electrode, and all three cannula portions define a fluid passageway between the distal tip and the interior fluid cavity of the handle unit, and mating the cannula module with the handle module.

In one embodiment, a multi-function surgical instrument is provided that includes a housing, a distal tip assembly, a waveguide conduit and at least one electrode. The housing has a proximal end and a distal end, and the housing includes a handle portion that forms the proximal end and a cannula portion that forms the distal end. The housing delimits an interior fluid cavity and a suction control port in fluid communication with the interior fluid cavity. The distal tip assembly is physically coupled to the cannula portion at the distal end of the housing, and the distal tip assembly includes a waveguide passage, a suction passage, and at least one electrode passage. The waveguide conduit extends from the waveguide passage of the distal tip through the cannula portion and into the handle portion. The at least one electrode extends from the at least one electrode passage of the distal tip through the cannula portion and into the handle portion.

In another embodiment, a multi-function surgical instrument is provided that includes a handle, a cannula assembly and at least one RF electrode. The handle has a proximal end and a distal end, and the handle includes: at least one interior fluid cavity, an exterior gripping surface, a fluid control port enabling fluid communication between an external environment and the interior fluid cavity, a waveguide passage that extends between the proximal end of the handle and the distal end of the handle, at least one RF electrode passage that extends between the proximal end of the handle and the distal end of the handle, and a suction passage that extends between the proximal end of the handle and the distal end of the handle. The cannula assembly includes a distal cannula portion with a distal tip, the distal tip including a distal tip suction passage, a distal tip waveguide passage, and at least one distal tip RF electrode passage, a proximal cannula portion physically coupled to the distal end of the handle, a central cannula portion between the distal cannula portion and the proximal cannula portion, a waveguide conduit that extends from the distal tip waveguide passage through the cannula assembly, the waveguide conduit aligned with the waveguide passage of the handle, and a fluid passageway that extends between the distal tip suction passage and the interior fluid cavity of the handle unit. The at least one RF electrode extends from the at last one distal tip RF electrode passage through the cannula assembly and through the at least one RF electrode passage of the handle.

In yet another embodiment, a method of manufacturing a multifunction surgical instrument is provided that includes: forming a first housing side-section having a proximal end and a distal end, the first housing side-section including a first portion of a waveguide lock receptacle and a first portion of a suction tubing connector receptacle; forming a second housing side-section having a proximal end and a distal end, the second housing side-section including a second portion of the waveguide lock receptacle and a second portion of the suction tubing connector receptacle; selecting a distal tip assembly that includes a waveguide passage, a suction passage, and at least one electrode passage; positioning a waveguide conduit in an interior portion of one of the first housing side-section and the second housing side-section, the waveguide conduit extending from the first portion of the waveguide lock receptacle to the distal end of the first housing side-section and into the waveguide passage of the distal tip assembly, or from the second portion of the waveguide lock receptacle to the distal end of the second housing side-section and into the waveguide passage of the distal tip assembly; positioning at least one electrode in the interior portion of the one of the first housing side-section and the second housing side-section, the at least one electrode extending from the proximal end to the distal end of the one of the first housing side-section and the second housing side-section, and extending at least partially through the at least one electrode passage of the distal tip assembly; and attaching the first housing side-section to the second housing side-section.

This disclosure further features a multi-function surgical instrument, comprising:
a handle having a proximal end and a distal end, the handle including:
   at least one interior fluid cavity,
   an exterior gripping surface,
   a fluid control port enabling fluid communication between an external environment and the interior fluid cavity,
   a waveguide passage that extends between the proximal end of the handle and the distal end of the handle,
   at least one RF electrode passage that extends between the proximal end of the handle and the distal end of the handle, and
   a suction passage that extends between the proximal end of the handle and the distal end of the handle; and
   a cannula assembly including:
a distal cannula portion with a distal tip, the distal tip including a distal tip suction passage, a distal tip waveguide passage, and at least one distal tip RF electrode passage,
a proximal cannula portion physically coupled to the distal end of the handle,
a central cannula portion between the distal cannula portion and the proximal cannula portion
a waveguide conduit that extends from the distal tip waveguide passage through the cannula assembly, the waveguide conduit aligned with the waveguide passage of the handle, and
a fluid passageway that extends between the distal tip suction passage and the interior fluid cavity of the handle unit; and
at least one RF electrode that extends from the at least one distal tip RF electrode passage through the cannula assembly and through the at least one RF electrode passage of the handle.

In certain embodiments, the handle is monolithic.

In certain embodiments, the handle is formed from at least two sections or shell pieces that, when assembled together, establish the exterior gripping surface, the interior fluid cavity and the fluid control port.

In certain embodiments, the multi-function surgical instrument comprises:
two RF electrodes, each of the two RF electrodes including a first portion positioned within the handle and a second portion positioned within the cannula assembly, the first portion and the second portion being electrically coupled to one another.

In certain embodiments, the handle includes at least one control switch that selectively controls at least one of RF energy delivery and optical radiation delivery.

In certain embodiments, the central portion of the cannula assembly is flexible.

In certain embodiments, the distal tip of the cannula assembly has a beveled distal surface, the beveled distal surface having an angle of zero degrees to sixty degrees relative to a reference line perpendicular to a longitudinal axis of the distal cannula portion.

In certain embodiments, the at least one RF electrode extends distally beyond the distal tip to a distance that provides a stand-off distance for laser energy directed through a waveguide, if any, in the distal tip waveguide passage.

In certain embodiments, the cannula module includes a first forceps arm and a second forceps arm, the first and the second forceps arms are mechanically joined to one another and are rotatable toward one another about a pivot point.

### BRIEF DESCRIPTION OF SEVERAL VIEWS OF THE DRAWINGS

In the figures, identical reference numbers identify similar elements or acts. The sizes and relative positions of elements in the figures are not necessarily drawn to scale. For example, the shapes of various elements and angles are not drawn to scale, and some of these elements are arbitrarily enlarged and positioned to improve legibility. Further, the particular shapes of the elements as drawn are not necessarily intended to convey any information regarding the actual shape of the particular elements and have been solely selected for ease of recognition in the figures.
FIG. 1 is a schematic perspective view of a multi-function surgical instrument (or "modular handpiece") according to one or more embodiments of the present disclosure having a proximal handle module and a distal cannula module for delivering laser and RF energy and with controllable suction shown in the "off" condition.
FIG. 2 is a side view of the handpiece of FIG. 1 showing the distal cannula module mated with the proximal handle module.
FIG. 3 is a cross-sectional view of the handpiece of FIG. 2.
FIG. 4A a schematic perspective, partially exploded view of the handpiece of FIGS. 1-3 without the handle unit.
FIG. 4B is a view similar to FIG. 4A with suction in the "on" condition.
FIG. 5A is an exploded perspective view of the handpiece of FIG. 1.
FIG. 5B is a view similar to FIG. 5A with proximal electrode tubes inserted into the proximal handle unit and with the distal components brought together to form the distal cannula module.
FIG. 5C is a schematic side view of an alternative modular handpiece according to the present disclosure carrying a waveguide and having a bend near its distal tip.
FIG. 5D is a side cross-sectional view of the handpiece of FIG. 5C.
FIG. 6 is a schematic perspective view similar to FIG. 5B showing an alternative elongated, partially bent distal cannula module suitable for surgery in airways.
FIG. 7 is a schematic, partially exploded view of an alternative modular handpiece according to the present disclosure having a proximal handle module and a distal cannula module including a unitary molded multi-lumen cannula with two distal RF electrodes and passages for a waveguide plus suction.
FIG. 8A is a back view of the handle unit of FIG. 7 prior to insertion of proximal components.
FIG. 8B is a front view of the modular handpiece of FIG. 7 after it has been fully assembled to have two distal electrode tips in the distal cannula module.
FIG. 9 is a schematic isometric cross-sectional view of the handle unit of FIGS. 7 and 8A.
FIG. 10A is a schematic isometric cross-sectional view of the distal cannula module of FIGS. 7 and 8B showing waveguide and suction passages formed in the cannula module, with a distal insert in the waveguide passage to serve as a stop.
FIG. 10B is an enlarged cross-sectional view of the distal insert of FIG. 10A.
FIG. 10C is a isometric view of the distal end of the cannula module of FIG. 8B and 10A-10B.
FIG. 11A is a schematic isometric cross-sectional view similar to FIG. 10A showing an alternative waveguide passage formed in the cannula module, with an integral waveguide stop established by a taper in the waveguide passage.
FIG. 11B is a view similar to FIG. 10C showing the distal end of the alternative cannula module of FIG. 11A.
FIG. 11C is a schematic side cross-sectional view of the handle module of FIG. 7 and the cannula module of FIGS. 11A and 11B showing interconnected waveguide and suction passages.
FIG. 12 is schematic perspective view of yet another modular handpiece according to the present disclosure having a push-button "hand-switch" energy controller in a handle module with a two-piece handle unit, and concentric electrodes in a distal cannula module with a "piggy-back" waveguide conduit.
FIG. 13 is a partially exploded view of FIG. 12.
FIG. 14 is a view similar to FIGS. 12 and 13 with the right-side half of the handle and suction control removed;
FIG. 15 is a schematic perspective view similar to FIGS. 12-14 with only the left-side handle and proximal fittings shown.
FIG. 16 is a side view of FIG. 14 with printed circuit board installed.
FIG. 17 is a schematic perspective view of the waveguide conduit and concentric electrodes of FIG. 16.
FIG. 18 is a front end view of the modular handpiece of FIG. 12.
FIGS. 19A-19C are schematic enlarged isometric views of alternative electrode tip configurations for the distal module of the handpiece of FIG. 12.
FIG. 20 is a schematic perspective view of still another modular handpiece according to the present disclosure that is similar to the handpiece of FIG. 12 but without push-button energy control.
FIG. 21 a schematic side partial-cross-sectional view of the handpiece of FIG. 20.
FIG. 22 is a schematic perspective view of yet another cannula module according to the present disclosure that includes forceps, shown in a manually closed condition.
FIGS. 23A and 23B are schematic perspective views of yet another novel, multifunction device including forceps, shown in the normally open and manually closed conditions, respectively.
FIG. 24 is a schematic perspective view of still another modular handpiece according to the present disclosure having a beveled suction and concentric electrode tip.
FIG. 25A is a schematic perspective view of a multi-function surgical instrument, in accordance with one or more embodiments, that includes a multi-piece injection molded housing and a distal tip assembly.
FIG. 25B is a schematic perspective view of the multi-function surgical instrument of FIG. 25A, with a right-hand section of the housing removed.

### DETAILED DESCRIPTION

In the following description, certain specific details are set forth in order to provide a thorough understanding of various disclosed embodiments. However, one skilled in the relevant art will recognize that embodiments may be practiced without one or more of these specific details, or with other methods, components, materials, etc. In other instances, well-known structures associated with handheld surgical instruments have not been shown or described in detail to avoid unnecessarily obscuring descriptions of the embodiments.

Unless the context requires otherwise, throughout the specification and claims which follow, the word "comprise" and variations thereof, such as, "comprises" and "comprising" are to be construed in an open, inclusive sense, that is as "including, but not limited to."

Reference throughout this specification to "one embodiment," or "an embodiment" means that a particular feature, structure, or characteristic may be combined in any suitable manner in one or more embodiments.

As used in this specification and the appended claims, the singular forms "a," "an," and "the" include plural referents unless the content clearly dictates otherwise. It should also be noted that the term "or" is generally employed in its sense including "and/or" unless the content clearly dictates otherwise.

The heads and Abstract of the Disclosure provided herein are for convenience are for convenience only and do not interpret the scope or meaning of the embodiments.

This disclosure provides, in one or more embodiments, a modular tool or handpiece that allows a user (e.g., physician) to apply suction, RF energy, and, or, optical energy during use, for example to tissue during a surgical procedure. The modular tool or handpiece has a proximal handle module with a handle unit having an exterior gripping surface sized, dimensioned and configured to be grasped by a hand of a user. The modular tool or handpiece includes at least one interior fluid cavity that extends therethrough. The modular tool or handpiece includes a fluid control port manipulable by a user to control fluid communication between the interior fluid cavity and the atmosphere external to the modular tool or handpiece, via which the user may perform fluid management, such as controlling an amount of suction at a tip of the modular tool or handpiece. The modular tool or handpiece includes structures sized and dimensioned to accommodate at least one waveguide for carrying optical radiation and capable of accommodating at least one RF electrode. The modular tool or handpiece also includes one or more circuit paths that extend therethrough, to couple RF energy to a pair of electrodes. The handpiece further includes a distal cannula module including a distal portion having a distal tip, a central portion, and a proximal portion connectable with the handle module. All three cannula portions are capable of at least one of (a) defining at least one waveguide conduit, (b) carrying at least one RF electrode, and (c) defining a fluid passageway between the distal tip and the interior fluid cavity of the handle unit, which preferably is connectable to a source of negative pressure for suction/evacuation and/or to a source of positive fluid pressure for irrigation.

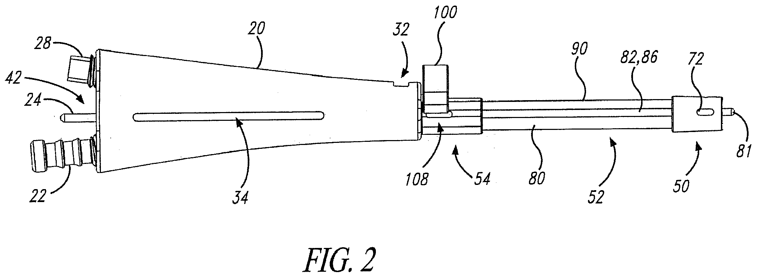

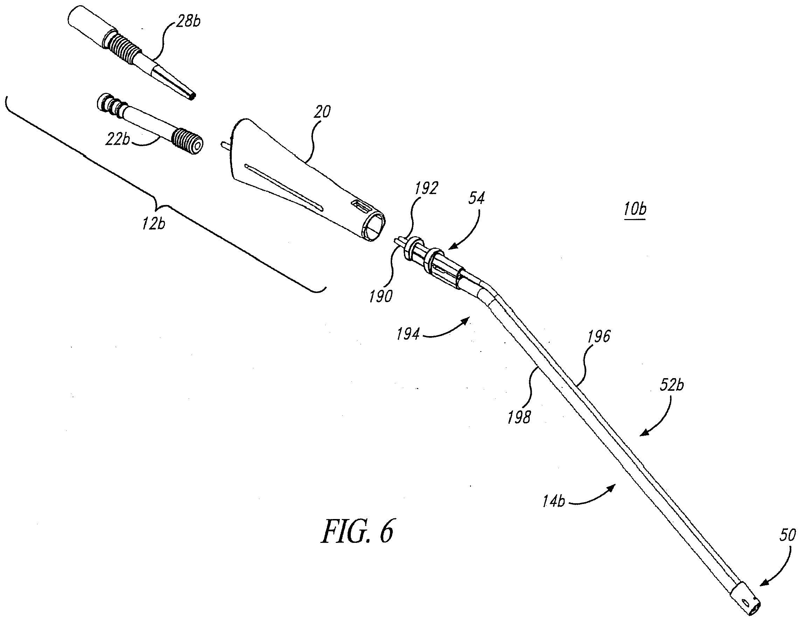

Multi-function surgical instrument 10 (which may be referred to herein as modular tool or handpiece 10) according to one or more embodiments of the present disclosure, as illustrated schematically in FIGS. 1-5B, includes a proximal handle module 12 and a distal cannula module 14, also referred to as a "cannula assembly". In some embodiments, at least a central portion of the cannula module 14 is flexible or bendable under forces likely to be experience when engaging bodily tissue during use , such as shown in FIG. 6 and described in more detail below.

Handle module 12, as shown in FIGS. 1-5B, includes a handle unit 20, a ribbed or barbed suction tubing connector 22 (also referred to as a hose fitting or a tubing coupler) connectable to a source of negative pressure, two proximal electrodes 24 and 26, and a waveguide lock 28 having a collet 29. In one or more embodiments, the handle unit 20 includes a distal opening 30, a suction control opening 32 (also referred to as a fluid control opening) that includes an edge 33, and at least one slot 34. The proximal end of handle unit 20 defines a proximal waveguide passage opening 40, two proximal electrode passage openings 42 and 44, and a suction passage opening 46 also referred to as a fluid passage opening.

In one or more embodiments, slot 34 assists formation of an interior electrode passage, between the proximal electrode passage opening 42 and the distal opening 30 of the handle unit 20, for electrode 24 during manufacture, for example, when handle unit 20 is monolithic and machined from a solid block of material, such as a polymer. As will be discussed in further detail below, in various alternatives, the handle unit 20 may advantageously comprise two or more injection molded pieces that are coupled or attached to one another, reducing the cost and waste associated with machining from a monolithic block. Additionally or alternatively, in one or more embodiments, the handle unit 20 may be formed by insert injection molding (or "overmolding"), in which one or more components in the handle unit 20 are inserted into a molding cavity and a molding material is injected into the cavity to form and solidify around the inserted components. For example, the electrodes 24, 26 (or an electrode passageway through which the electrodes 24, 26 may be passed), one or more waveguide passages (e.g., waveguide passages 151, 152, 154, 156) and one or more suction or fluid passages (e.g., suction passages 122, 124, 126) may be provided as components that inserted into a molding cavity, and the handle unit 20 may be formed by injection molding to surround the inserted components.

A similar slot (not shown) assists formation of an interior electrode passage, between the proximal electrode passage opening 44 and the distal opening 30, for the other electrode 26. Opposing holes are drilled at proximal and distal locations 36 and 38, and then slot 34 is formed therebetween. Additional holes are then drilled at the proximal and distal (if needed) ends of handle unit 20 to complete the electrode passages to enable subsequent insertion of the proximal electrodes 24 and 26 into the handle unit 20.

Cannula module 14 includes a distal portion 50 having a distal tip 60, a central portion 52, and a proximal portion 54 that is connectable with the handle module 12 (for example, by friction fit) when inserted within distal opening 30 of the handle unit 20. All three cannula portions (i.e., distal portion 50, central portion 52, and proximal portion 54) together define a waveguide conduit, are capable of carrying at least one RF electrode, and define a fluid passageway (e.g., for suction) between the distal tip 60 and an interior fluid cavity 128 of the handle unit 20. The interior fluid cavity 128 (FIG. 3) selectively communicates with an external environment (e.g., the atmosphere) via port 32 as described in more detail below.

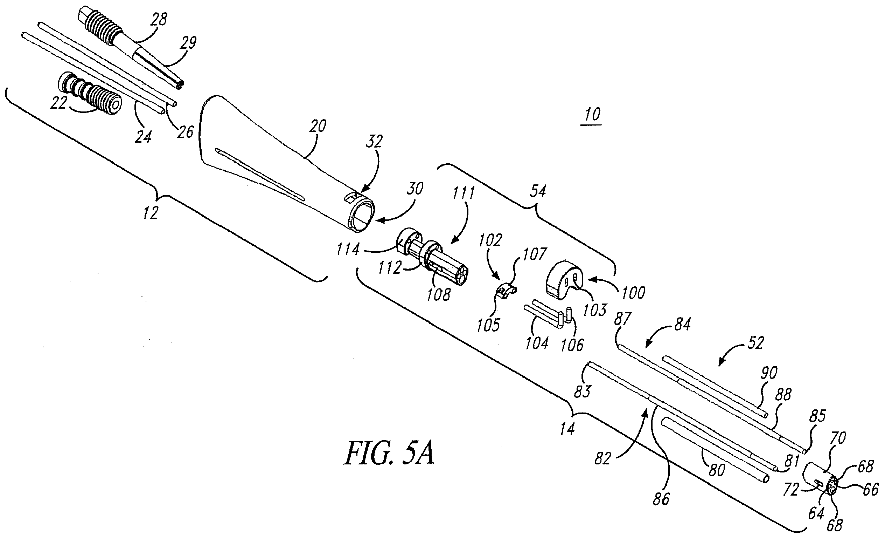

As seen most easily in FIG. 5A, distal portion 50 of cannula module 14 defines a waveguide distal passage 62, two electrode distal passages 64 and 66, and a suction distal passage 68. Distal portion 50 also defines at least one side slot 72, in one or more embodiments, to assist formation of electrode distal passage 64. In one or more embodiments, a similar slot 74 (not shown) is formed on the opposing side of distal portion 50 for assisting formation of distal passage 66.

Central cannula portion 52 may be bendable, as described in more detail below in relation to FIG. 6, and includes a suction conduit 80 (FIGS. 1-5B) a waveguide conduit 90, and two electrodes 82 and 84 having electrically non-conducting insulation 86 and 88, respectively, covering portions of the electrodes 82, 84. As most easily seen in FIG. 5A, electrode 82 has electrically-exposed distal and proximal ends 81 and 83, respectively, and electrode 84 has electrically-exposed distal and proximal ends 85 and 87, respectively. A shorter-length central portion 52 such as shown in FIGS. 1-5B is most suited for more shallow surgeries such as oral cavity surgeries. However, the central portion 52 may have any length as may be desirable for use in other types of surgeries, including, for example, laparoscopic surgeries.

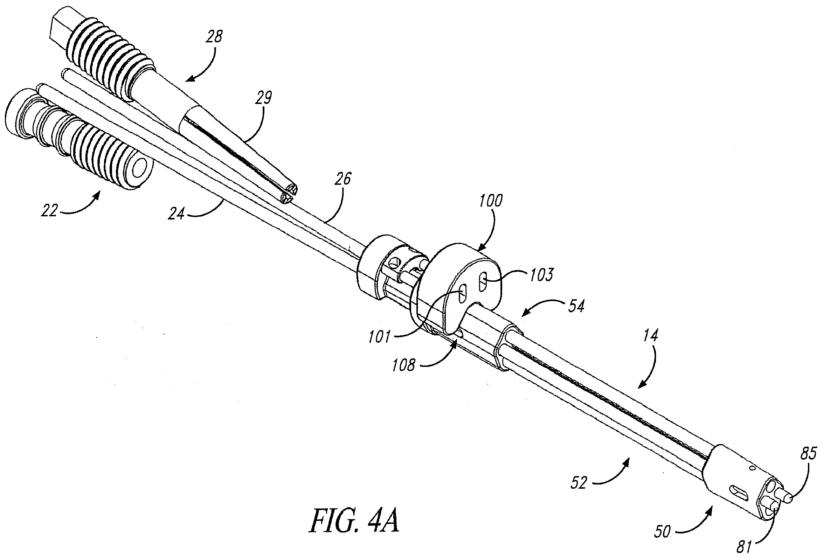

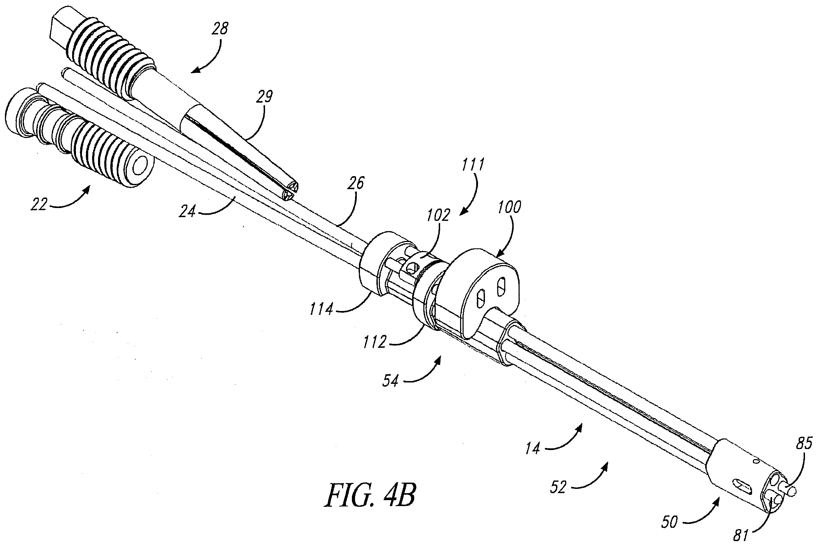

Proximal cannula portion 54 includes a slideable fluid control trigger 100 that is attached to the distal ends of rails 104 and 106, such as by placing an adhesive or set screws through openings 101 and 103. In one or more embodiments, trigger 100 includes tabs or other projections which slidably engage one or more slots in multi-lumen cannula unit 111 such as slot 108. Slideable suction control cover 102 is attached to a proximal portion of rails 104 and 106 by set screws placed through holes 105 and 107, respectively, after the rails are passed through corresponding pairs of holes in enlarged ribs 112 and 114 of multi-lumen cannula unit 111. During use, a user typically grasps the exterior surface of handle unit 20 with the palm and fingers of one hand, and then utilizes a thumb or finger to control suction utilizing trigger 100. Movement by a user of the trigger 100 from a proximal position shown in FIGS. 1-4A to a distal position shown in FIG. 4B pulls the cover 102 distally via rails 104 and 106 which thereby blocks exposure of interior cavity 128 to the atmosphere via port 32 and changes the condition of handpiece 10 from "suction off" to "suction on" such as shown in FIG. 4B. Alternative embodiments may include single or triple rails, tracks or similar slideable control mechanisms.

As shown in FIG. 3, a suction passage 120 is established between the suction tubing connector 22 and the opening at the distal tip of the suction distal passage 68 by suction passages 122, 124, and 126 defined by (or provided within) handle unit 20, and by an internal fluid cavity 128 defined by (or provided within) both the handle unit 20 and multi-lumen cannula unit 111 which enables fluid communication with the atmosphere via port 32 when not blocked by cover 102. The inner walls of suction passage 122 define threads, which mate with threads of suction tubing connector 22. In one or more embodiments, the perimeter dimension (e.g., diameters) of suction passages 122, 124, 126 and cavity 128 become progressively smaller (along a proximal-to-distal direction) as illustrated. Multi-lumen cannula unit 111 further defines a fluid or suction passage 130 which communicates with suction passage 132 within suction conduit 80. The distal end of the suction conduit 80 mates with the suction distal passage 68 within the distal tip portion 50.

As also shown in FIG. 3, a waveguide passage 150 is established proximally by central lumen 27 within waveguide lock 28, which threadably engages passage 151 in handle unit 20. The waveguide passage 150 extends between the proximal end of the waveguide lock 28 and the opening at the distal tip of the waveguide distal passage 62 in the distal portion 50 of the cannula module 14. Proceeding distally, handle unit 20 further defines waveguide passages 152, 154 and 156 which communicate with a waveguide passage 158 of multi-lumen cannula unit 111. In one or more embodiments, the waveguide passage 154 has tapered walls with an interior perimeter dimension progressive decreasing along the proximal-to-distal direction, and which force the collet 29 of the waveguide lock 28 to press on a waveguide, when inserted through the waveguide passage 150, as the collet 29 advances distally in the tapered waveguide passage 154, e.g., as described by Shurgalin et al. in U.S. Patent Publication No. 2014/0316395A1. Waveguide passage 158 of the multi-lumen cannula unit 111 communicates with a waveguide lumen 160 of the waveguide conduit 90, which communicates with the waveguide distal passage 62 in the distal portion 50 of the cannula module 14, which is open at its distal end.

In one or more embodiments, distal tip portion 50 further defines a "built-in" waveguide stop 57, which is created by the waveguide distal passage 62 having a narrower inner perimeter dimension (e.g., diameter) slightly distal to the waveguide sight hole 70. The inner perimeter dimension of the waveguide distal passage 62 may be tapered from a wider dimension to a narrower dimension, which forms the waveguide stop 57. Alternatively, the waveguide distal passage 62 may include two separate portions, each having a substantially continuous inner perimeter dimension, e.g., a wider portion (e.g., at the proximal end of the distal tip portion 50) having a first inner perimeter dimension, and a narrower portion (e.g., distal to the wider portion) having a second inner perimeter dimension that is smaller than the first inner perimeter dimension. The waveguide stop 57 may thus be formed at the interface between the wider and narrower inner perimeter dimensions, and the narrower inner perimeter dimension may be sized to prevent a waveguide inserted through the waveguide passage 150 from advancing beyond the waveguide stop 57.

The inner wall defining the waveguide distal passage 62 then enlarges distally (from the waveguide stop 57 to the opening at the distal tip of the passage 62), which accommodates a laser beam that has an increasingly larger diameter as the beam emerges from the distal tip of the waveguide. The taper leading to the waveguide stop 57 also serves to create a nozzle effect for gas that may be forced through the waveguide to cool the waveguide and clear bodily fluid from the opening at the distal tip of the waveguide distal passage 62. For example, a helium gas is typically delivered through a hollow waveguide at 70 psi to 90 psi (pounds per square inch). In one or more embodiments, a waveguide stop is not included, which enables advancement of the waveguide beyond the distal tip 60 of the distal portion 50.

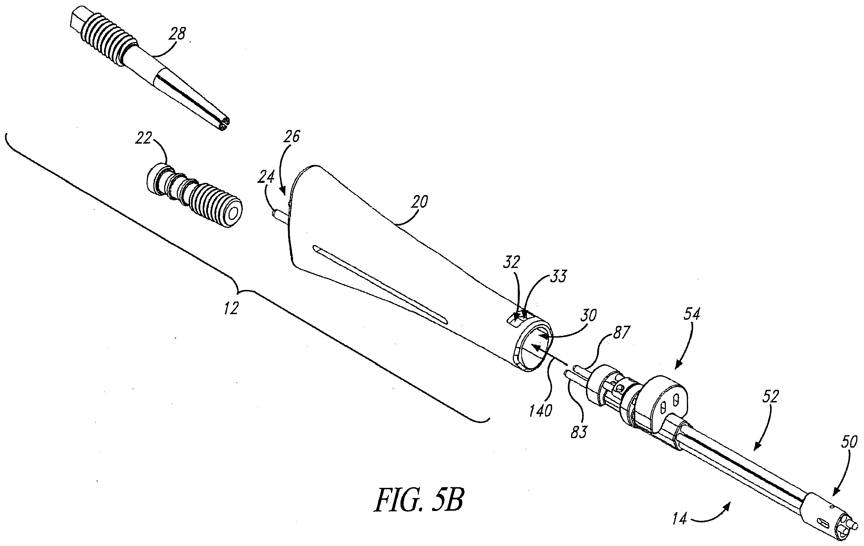

In one or more embodiments, at least the distal ends of proximal electrodes 24 and 26 are hollow, and proximal ends 83 and 87 of distal electrodes 82 and 84 are insertable into proximal electrodes 24 and 26 (FIG. 4B) when cannula module 14 is inserted into distal opening 30 in handle unit 20 such as indicated by arrow 140 in FIG. 5B. When bendability of the central portion 52 is desired, the distal electrodes 82 and 84 may have a diameter within a range of 1 mm, inclusive, to 2mm, inclusive. The distal electrodes 82 and 84 are positioned extending through the electrode distal passages 64 and 68, respectively, with the distal ends 81 and 85 of distal electrodes 82 and 84 extending beyond the distal tip 60 to a distance up to about 3mm, and may preferably be within a range of 2mm, inclusive, to 3mm, inclusive. The distal ends 81 and 85 of distal electrodes 82 and 84 thus define a "standoff" distance, which is the distance between the distal tip of the distal portion 50. The standoff distance may be selected to have any distance suitable to prevent bodily tissue from being sucked into or from blocking the opening at the suction distal passage 68. Additionally, the distal ends 81 and 85 of distal electrodes 82 and 84 may have a spacing between them of up to 3mm. Suitable electrode materials include copper, silver, palladium, titanium and gold, as pure metals or as alloys, including stainless steel alloys. When RF energy is desired, a standard RF connector is engaged with the proximal ends of proximal electrodes 24 and 26. Typically, a foot-operated switch is utilized by a user to energize the electrodes as desired.

During assembly of cannula module 14, the distal electrodes 82 and 84 plug into the proximal electrodes 24 and 26, and the enlarged ribs 112 and 114 of the multi-lumen cannula unit 111 (which may also referred to herein as an "endcap") of the proximal portion 54 mates (e.g., by a frictional fit) with the inner body of the handle unit 20, forming a connection between the two parts such as shown in FIG. 3. The waveguide passage 150 in the handle unit 12 aligns with the waveguide lumen 160 in the cannula module 14 and the suction passage 120 in the handle unit 20 aligns with the suction passage132 in the cannula module 14.

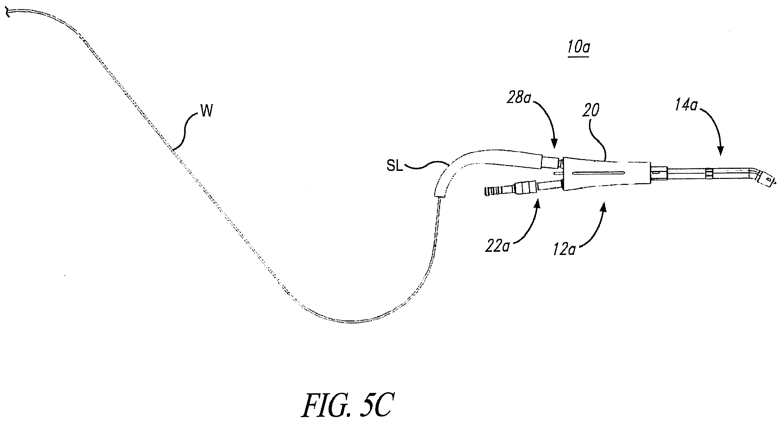

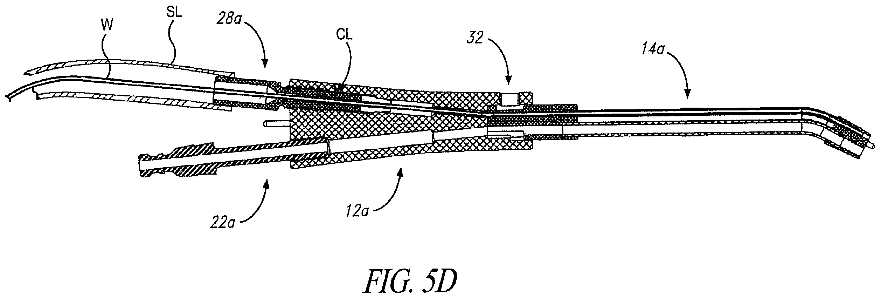

An alternative modular handpiece 10a according to the present disclosure (shown in FIGS. 5C and 5D) carries a waveguide W supported by a strain relief support sleeve SL that removably attaches to waveguide lock fitting 28a including a collet CL inside handle module 12a. Waveguide locks with collets are described by Shurgalin et al. in U.S. Patent Publication No. 2014/0316395 A1. An alternative suction tube connector 22a accommodates different diameters of suction tubing. Cannula module 14a has a bend near its distal tip.

FIG. 6 is a schematic perspective view similar to FIG. 5B showing a modular handpiece 10b with an alternative elongated, partially bent distal cannula module 14b suitable for surgery in airways. Handle module 12b differs from handle module 12 of FIGS. 1-5B in that an alternative waveguide lock 28b and a different suction connector 22b are utilized in this construction.

The handpiece 10b shown in FIG. 6 may be particularly suitable for use in airway surgeries, as the cannula module 14b includes an elongated central portion 52b. Waveguide conduit 196 and suction conduit 198 include at least one bend region 194 which is an elbow with a fixed bend angle in some embodiments and, in other embodiments, is a flexible or bendable section. As a separate observation, there are no distal electrodes in this construction of handpiece 10b, which demonstrates an embodiment with laser and suction functionality, and that uses proximal electrode pins 190 and 192 as a mechanism for connecting the cannula module 14b to the handle module 12b. The bend angles shown in FIG. 6 are not shown as having preferred radii of bend for most airway surgeries, but are depicted as shown for ease of illustration.

Modular handpieces according to the present disclosure can be considered as versatile platforms which readily enable different cannula modules to be assembled with and insertable into a given handpiece module. Each cannula module can have a selected length and other dimensions that are suitable for an intended procedure.

Further, any number of functions can be incorporated into handpieces according to the present disclosure. For example, distal electrodes are shortened or omitted from handpiece 10b (FIG. 6) when only laser and suction capabilities are needed for certain intended surgical procedures. Other combinations of functionality provided by handpieces according to various embodiments of the disclosure include, for example: (a) only laser and bipolar RF energies; (b) only suction and bipolar RF energy, without a waveguide; (c) laser only, in which case the suction tube connector can also be omitted; (d) suction only, without a waveguide or distal electrodes; and (e) bipolar RF energy only. While various embodiments are described herein with respect to bipolar RF functionality, embodiments provided herein are not limited to such functionality. For example, monopolar RF energy may be utilized in place of bipolar RF energy in alternative embodiments.

Suitable materials for the polymeric components of handpieces 10, 10a, and 10b include those listed as follows, per component. Handle/Cannula Endcap/Cannula Distal Tip: Polymeric materials from one or more of PEEK, Ultem®, Radel® families, Polycarbonates, Polyesters, Polyurethanes, and UHMW (Ultra-High Molecular Weight) polyolefins. Electrodes: copper, silver, palladium and gold based alloys or pure metals, titanium, stainless steel. Waveguide Cannula: Polymer materials from PEEK, Ultem®, Radel® families, Polycarbonates, Polyesters, Polyurethanes and UHMW polyolefins, Stainless Steel, and Aluminum. Suction Cannula: Polymer materials from PEEK, Ultem®, Radel® families, Polycarbonates, Polyesters, Polyurethanes and UHMW polyolefin, Silicone, Stainless Steel, Aluminum.

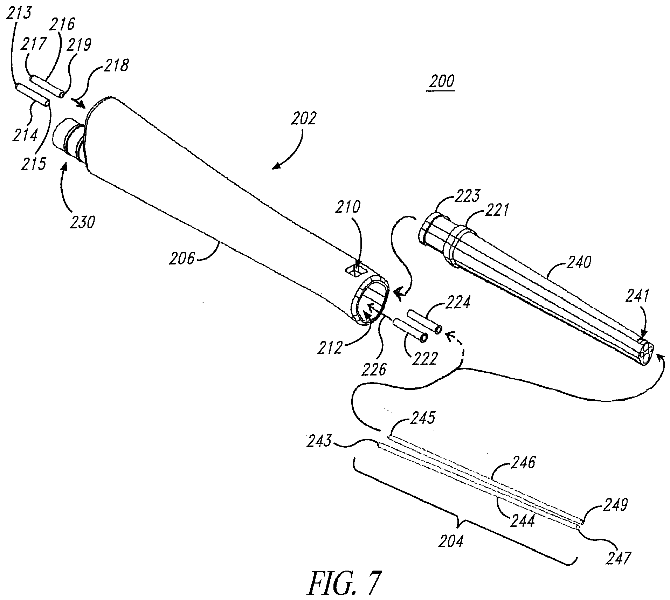

An alternative multi-function or modular handpiece 200 according to one or more embodiments of the present disclosure, as shown in FIGS. 7-10C, has a proximal handle module 202 and a distal cannula module 204. Handle module 202 includes a handle unit 206 defining a suction port 210 and a distal opening 212. During use, a user typically grasps the exterior surface of handle unit 206 with the palm and fingers of one hand, and then utilizes a thumb or a finger to cover the suction port 210 when suction is desired at the distal end of cannula module 204 (e.g., at the distal end of the suction passage 270). Two proximal electrode connector pins 214 and 216 are insertable into the proximal end of handle unit 206 as indicated by arrow 218. The proximal electrode connector pins 214 and 216 may be generally hollow with rounded proximal ends 213 and 217 and distal openings 215 and 219. Handle module 202 also includes ferrules 222 and 224 having an outside diameter at their proximal ends which is slightly smaller than the diameter of openings 215 and 219 of proximal electrode connector pins 214 and 216. During manufacture of handle module 202, ferrules 222 and 224 are inserted through distal opening 212 as indicated by arrow 226 to electrically connect with proximal electrode connector pins 214 and 216. The electrical connection is made by mechanically connecting them in one construction and, in another construction, by connecting them with intermediate electrically conducting elements which may extend through the handle unit 206. The proximal end of handle unit 206 further includes a suction tube connector or integral hose fitting 230 which is connectable to suction tubing in fluid communication with a source of negative pressure (not shown).

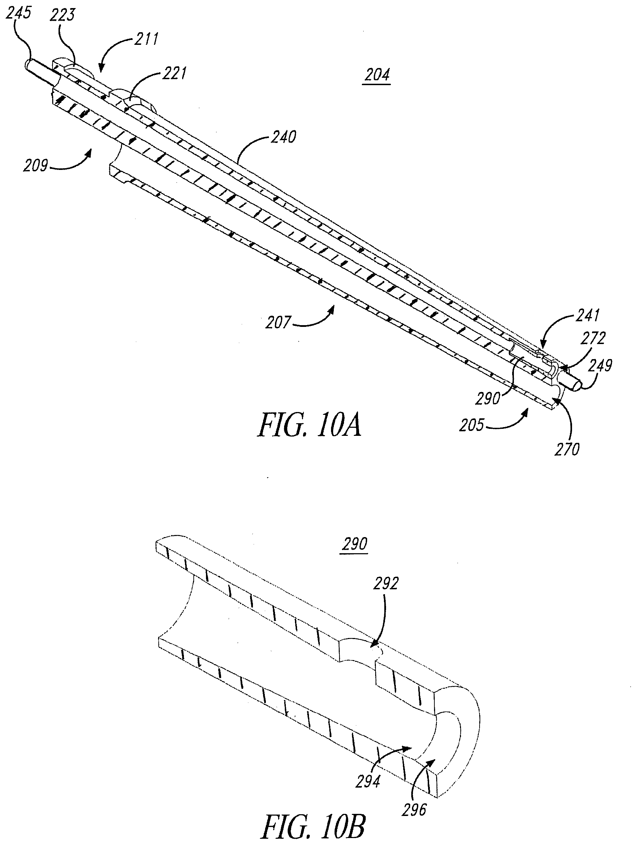

Cannula module 204 includes a unitary molded multi-lumen distal cannula unit 240 with two RF electrodes 244 and 246 carried in separate passages and having proximal ends 243 and 245 which are mechanically and electrically connectable with ferrules 222 and 224. Cannula unit 240 defines also a waveguide passage 272 (shown in FIGS. 10A-10C) for a waveguide, as well as a suction passage 270.

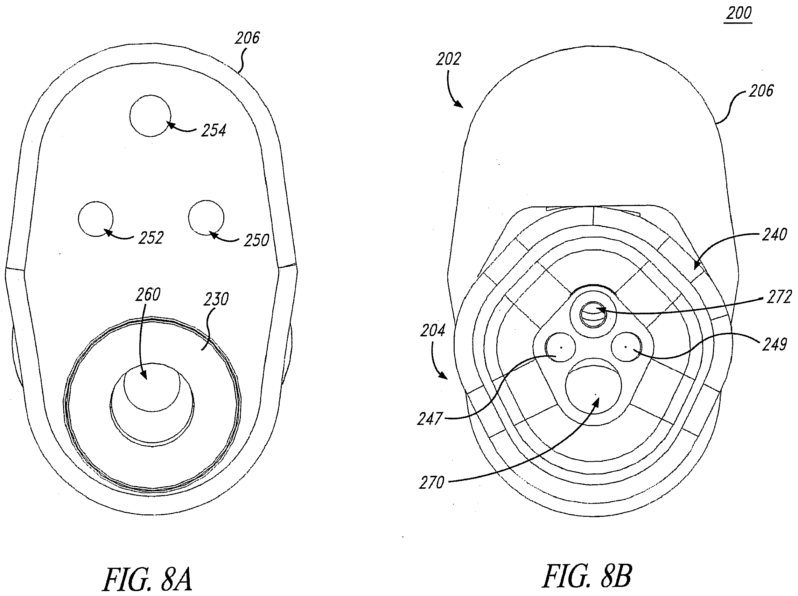

FIG. 8A is a back view (i.e. facing the proximal end) of the handle unit 206 prior to insertion of proximal components (e.g., the hose fitting 230, and the proximal electrode connector pins 214, 216). Electrode pin openings 250 and 252 receive pins 214 and 216, respectively, which are electrically connected to ferrules 222 and 224, respectively, either directly or via intermediate electrically conducting elements through the handle unit 206. Waveguide opening 254 receives a waveguide lock, which may be threadably engageable to the handle unit 206 via threads formed in waveguide opening 254, and a waveguide may be passed through an opening in the waveguide lock, as previously described herein. Suction passage 260 is shown within hose fitting 230. FIG. 8B is a front view (i.e. facing the distal end) of the handpiece 200 of FIG. 7 after it has been fully assembled to have two distal electrode tips 247 and 249 projecting from the cannula unit 240 of cannula module 204. An opening at the distal end of the suction passage 270 is also shown, as is an opening at the distal end of the waveguide passage 272.

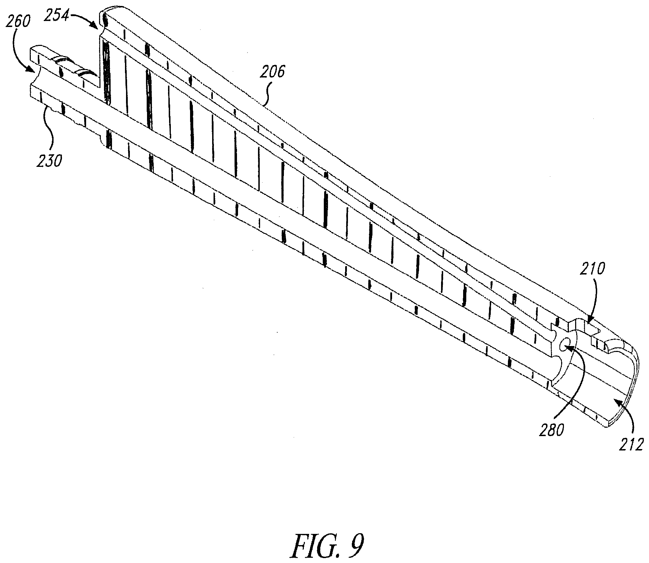

FIG. 9 is a schematic isometric cross-sectional view of the handle unit 206 of FIGS. 7 and 8A. Distal passage 280 for receiving ferrule 224 is visible, as well as waveguide passage 254 and suction passage 260 which is in fluid communication with suction port 210 after handpiece 200 is fully assembled.

FIG. 10A is a schematic isometric cross-sectional view of the cannula module 204 of FIGS. 7 and 8B, which includes distal portion 205, central portion 207, and proximal portion 209 that includes ribs 221 and 223. The proximal portion 209 also defines a channel or gap 211 which enables communication of the internal fluid cavity with a suction control port of a handle module. That is, the gap 211, when inserted into a handle module, is spaced apart from inner walls of the handle module along a circumference of the cannula module 204 between the ribs 221 and 223. The gap 211 is in fluid communication with an internal fluid cavity (e.g., internal fluid cavity 128 shown in FIG. 3), as well as with a suction control port (e.g., port 32 of FIG. 3) of the handle module. Accordingly, control of a suction force at the distal tip of the cannula module (e.g., at the distal end of the suction passage 270) may be accomplished by selectively opening and closing (e.g., by a user's thumb or finger, or by a mechanical feature such as a slideable cover, as described herein) the suction control port. In that regard, it will be noted that a size of the suction control port may preferably be larger than a size of the distal end of the suction passage 270 for better control of the suction force at the distal end of the suction passage 270.

Also shown in FIG. 10A are waveguide passage 272 and suction passage 270 formed in the cannula unit 240. A distal insert 290 in the waveguide passage 272 includes or otherwise forms a waveguide stop 294 (FIG. 10B), which prevents a waveguide inserted into handpiece 200 from advancing distally past the waveguide stop 294. The waveguide stop 294 may be the same as, or similar to, the waveguide stop 57 described with respect to FIG. 3, with a tapered inner perimeter dimension or with a stepped decrease from a wider inner perimeter dimension to a narrower inner perimeter dimension. In one or more embodiments, the waveguide stop 294 (or any waveguide stop as described with respect to any of the embodiments herein) may include or otherwise be physically coupled to a locking mechanism adjacent to the waveguide stop, which holds the waveguide in its desired position (e.g., abutting the waveguide stop) and prevents the waveguide from being pulled out of the waveguide stop. For example, the locking mechanism may be a spring-biased collet that pushes against the waveguide and holds the waveguide in its desired position. The locking mechanism in the waveguide stop may be releasable in some forms (e.g., by a button or similar manually-actuatable mechanism coupled to the spring-biased collet, which, when actuated, releases the spring force acting against the waveguide), or may be un-releasable in other forms.

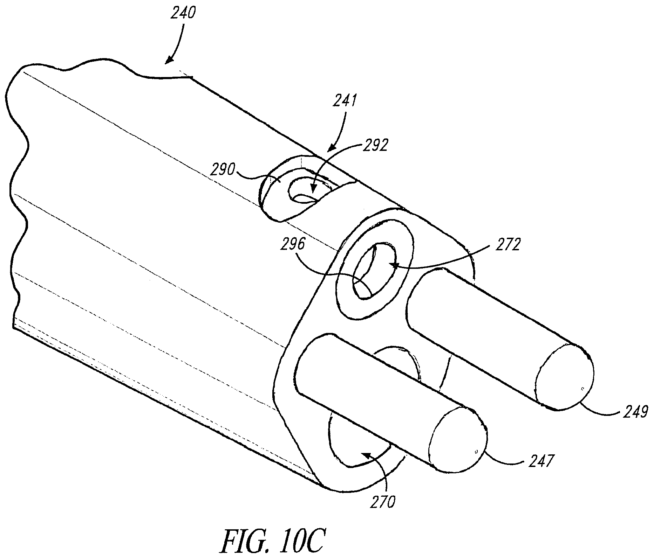

FIG. 10B is an enlarged cross-sectional view of the distal insert 290 of FIG. 10A showing a tapered inner diameter leading distally to stop 294, with a flare 296 to accommodate a diverging beam of optical radiation emitted from the waveguide. FIG. 10C is a isometric view of the distal end of the cannula module of FIG. 8B and 10A-10B. The distal end may include a sight hole 241, and the distal insert 290 may include a corresponding distal insert sight hole 292 that is aligned with the sight hole 241. When a waveguide is inserted in the cannula unit 240 and advanced distally to, or near, the stop 294 in the distal insert 290, the waveguide may be viewable through the sight holes 241 and 292. By viewing the waveguide through the sight holes 241, 292, a user may visually confirm the positioning of the waveguide at the stop 294.

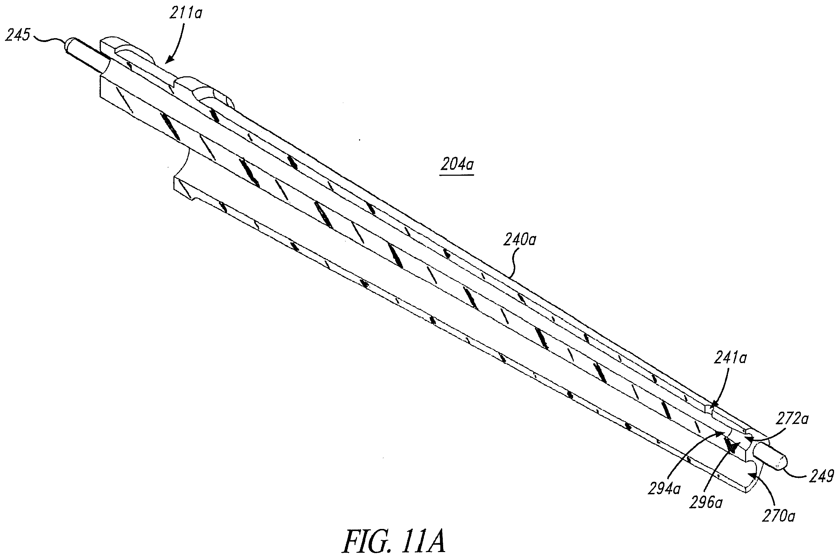

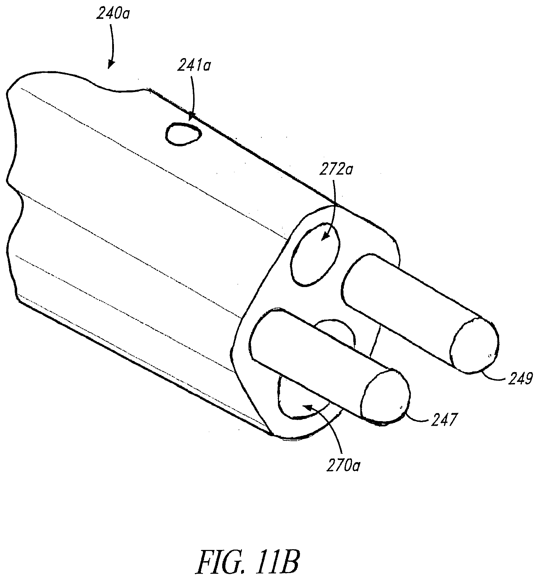

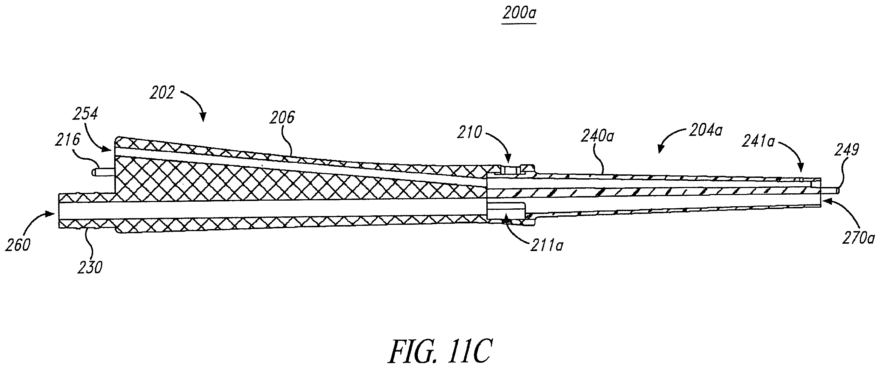

FIG. 11A is a schematic isometric cross-sectional view similar to FIG. 10A showing an alternative waveguide passage 272a formed in the cannula 240a, with an integral waveguide stop 294a established by a taper in the waveguide passage 272a, ending distally in a flare 296a. The embodiment shown in FIGS. 11A-11C is substantially the same as that shown in FIGS. 10A-C, except that a distal insert is not included in the embodiment shown in FIGS. 11A-11C. Instead, the waveguide stop 294a and flare 296a are integrally formed in the cannula 240a of FIGS. 11A-11C. Sight hole 241 a enables viewing of a waveguide when its distal end reaches stop 294a, and allows a user to visually confirm the positioning of the waveguide. A suction passage 270a is formed in the cannula 240a. Fluid recess channel or gap 211 a enables fluid communication with suction port 210, as shown in FIG. 11C. FIG. 11B is a view similar to FIG. 10C showing the distal end of the alternative cannula module 204a of FIG. 11A, also with electrode tips 247 and 249, which can serve as "stand-offs" or aiming guides for laser beams or other non-visible surgical energy delivery. FIG. 11C is a schematic side cross-sectional view of the handle module 202 of FIG. 7 and the cannula module 240a of FIGS. 11A and 11B showing interconnected waveguide and suction passages.

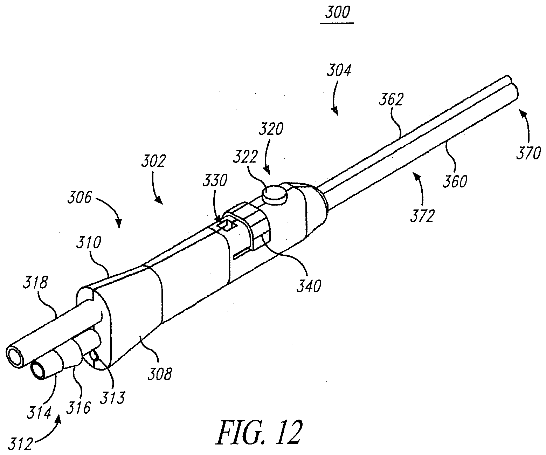

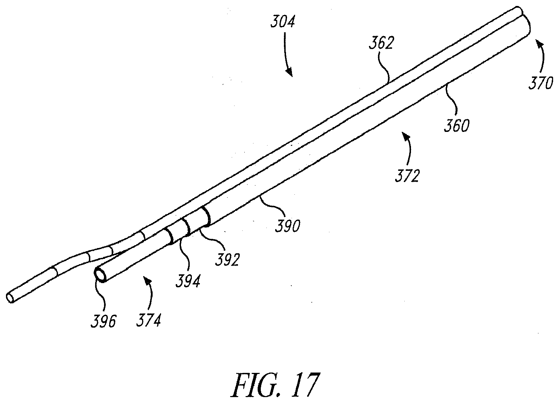

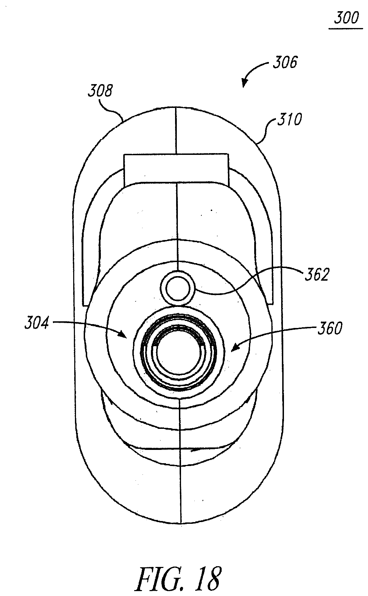

Yet another multi-function or modular handpiece 300 according to one or more embodiments of the present disclosure, shown in FIGS. 12-21 and FIG. 24, includes a push-button-activated, "hand-switch" energy controller 320 in a handle module 302 with a two-piece handle unit 306, and concentric electrodes in a distal cannula module 304 with a waveguide conduit 362. Handle unit 306 includes a right-side section 308 and a left-side section 310, also referred to as shells or shell pieces, which together form an exterior gripping surface capable of being grasped by the hand of a user. Each of the right-side section 308 and the left-side section 310 may be formed, for example, by injection molding. The handpiece may then be assembled by placing the various components (which will be described in further detail below) within one or both of the right-side section 308 and the left-side section 310, and attaching the right-side section 308 and the left-side section 310 to one another, for instance via heat welding or RF welding, and, or via adhesive or epoxy.

A ribbed or barbed suction tubing connector or hose fitting 312 is provided at a proximal end of the handle unit 306, and has a first, smaller-diameter barb 314 and a second, larger-diameter barb 316 to enable the hose fitting 312 to accommodate and to provide a fluid-tight seal when connected to various sizes or diameters of suction hoses.

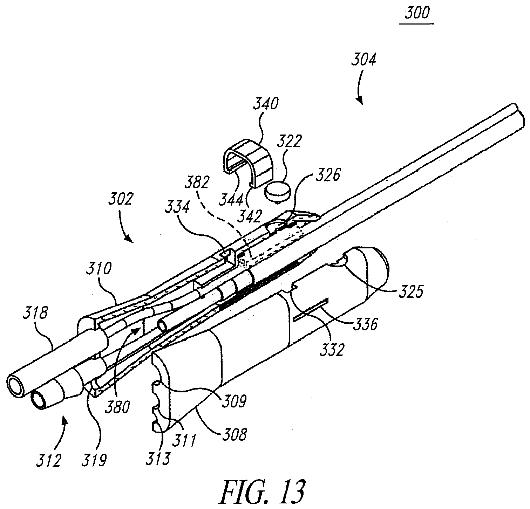

Handle module 302 further includes a waveguide lock 318 positioned above hose fitting 312, in in one or more embodiments. Handle sections 308 and 310 define openings 309 and 315 to accommodate waveguide lock 318, define openings 311 and 317 to accommodate fitting 312, and define openings 313 and 319, respectively, to accommodate control wiring and/or wiring to carry RF current to selectively energize electrodes as described in more detail below. After components are placed into one or both of handle sections 308 and 310, such as shown and described below in relation to FIGS. 13-16, then the matching edges of sections 308 and 310 are mated and secured together with an adhesive such as an epoxy, or by any plastic welding technique, such as heating, ultrasonic welding, RF welding, or the like, or by any other bonding technique.

A suction port 330 is established by openings 332 and 334 in handle sections 308 and 310, respectively, after the sections 308 and 310 are secured together. A suction control cover 340 has tabs 342 and 344 which slidably engage at least one slot 336 formed in at least one of the handle sections, and shown in FIGS. 12 and 13 as being formed in handle section 308. During use, a user typically grasps the exterior surface of handle unit 306 with the palm and fingers of one hand, and then utilizes a thumb or finger to control suction by covering port 330 directly with the thumb or finger, or by moving suction control cover 340 proximally to block suction port 330. Handpiece 300 is shown in the "suction off" condition in FIG. 12 with suction port 330 open, which fully exposes interior fluid cavity 380 to the external atmosphere.

By forming the handle unit 306 as two pieces, i.e. right-side section 308 and left-side section 310, the interior fluid cavity 380 may have a larger volume (e.g., as compared to a machined, monolithic handle unit) and may be formed by attaching the molded sections 308, 310 to one another, the inner walls of which define boundaries of the interior fluid cavity 380. Thus, a separate suction passage or suction conduit does not need to be provided through the handle unit 306, since the entire interior fluid cavity 380 may be utilized as a suction passage.

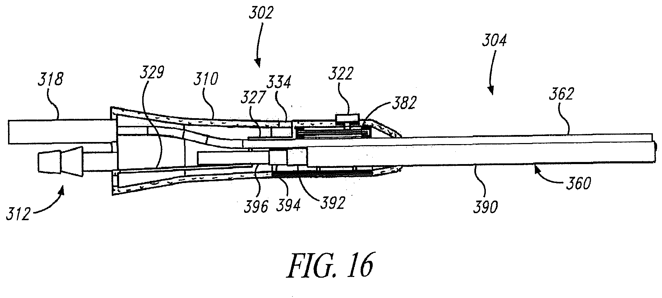

Energy controller 320 is a type of physically-actuatable switch, which in one or more embodiments includes button 322 capable of being actuated by a finger or thumb of a user to control at least one of RF energy delivery and optical radiation delivery. When depressed, button 322 is driven into recesses 325 and 326 of handle sections 308 and 310, respectively, and a lower projection extending below button 322 pushes against an electrical contact, such as a snap-dome, on printed circuit board 382 (FIG. 16), which is shown in phantom in FIGS. 13-14. The normally open switch 320 is thereby closed to complete an electrical control circuit extending proximally through openings 313, 319 to an RF generator, a laser, or other source of surgical energy.

Push-button-activated control of RF energy, insufflation and other functions, such as for hand-held electrosurgical pencils, is described by Sartor in U.S. Patent No. 7,828,794, for example. The term "switch" as utilized herein has a similarly broad meaning to include "electrical actuators, mechanical actuators, electro-mechanical actuators (rotatable actuators, pivotable actuators, toggle-like actuators, buttons, etc.) or optical actuators." Cannula module 304 includes a combined electrode and suction cannula 360 and a smaller-diameter waveguide conduit 362 which, in one or more embodiments, is secured to at least a portion of cannula 360 by an adhesive, with the waveguide conduit 362 positioned above the cannula 360. Cannula module 304 has a distal portion 370, a central portion 372, and a proximal portion 374 that is positioned wholly inside handle unit 306 in one or more embodiments.

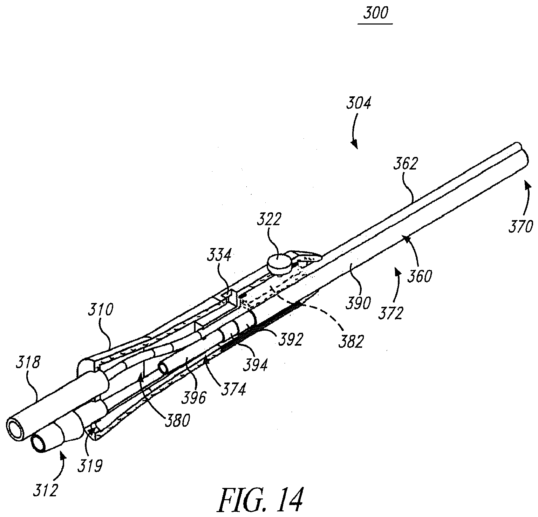

Cannula 360 is shown in more detail in FIGS. 14, 16 and 17 having an outer insulation tube or jacket 390, an outer electrode 392, an inner insulation tube 394, and an inner electrode 396. In other words, electrodes 392 and 396 are concentric with each other in this construction, separated by one or more electrically non-conducting layers.

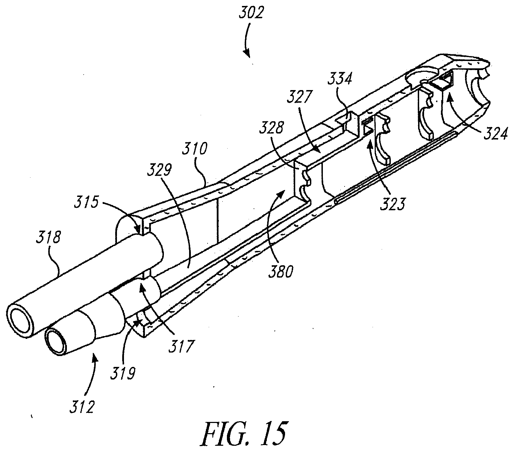

FIG. 15 is a schematic perspective view similar to FIGS. 12-14 with only the left-side handle section 310 and proximal hose fitting 312 and waveguide lock 318 shown during initial assembly of handpiece 300. Molded shelves 323 and 324 support the printed circuit board 382 (FIG. 16), and securely hold the printed circuit board 382 in a desired position with respect to the button 322. Molded barriers 327, 328 and 329 support one or more components such as cannula 360 and portions of waveguide conduit 362, and further define interior fluid cavity 380 when mated with corresponding barriers in handpiece section 308.

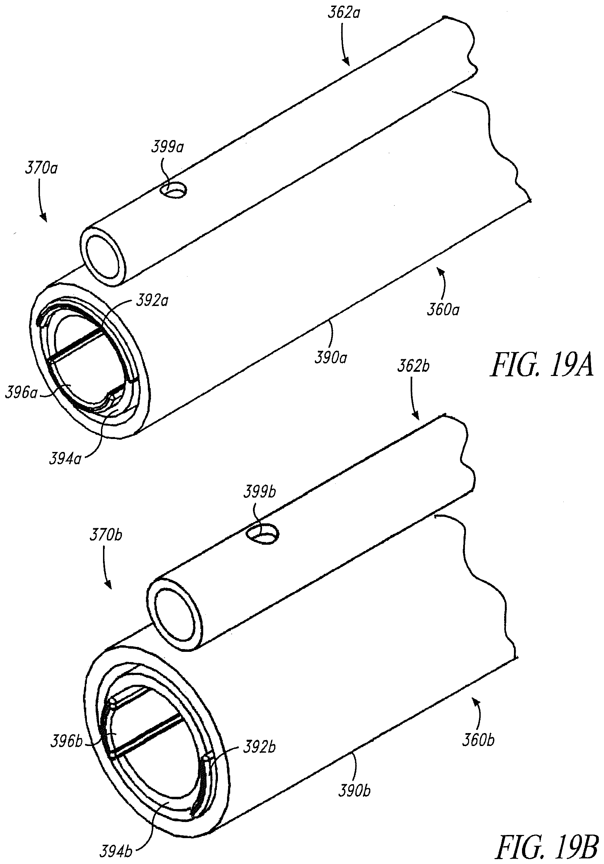

FIGS. 19A-19C are schematic enlarged isometric views of alternative electrode tip configurations for the distal portion 370 of cannula module 304 of the handpiece 300 of FIG. 12. As shown in FIG. 19A, the cannula module 304 may have a distal portion 370a in which circular parallel electrodes 392a and 396a have semi-circular distal tips that are offset (i.e., spaced apart) from each other vertically in relation to waveguide conduit 362a and are separated by insulation tube 394a. Waveguide conduit 362a is shown with a sight hole 399a. Additionally, the semi-circular distal tips of electrodes 392a and 396a may extend distally beyond the distal tip of waveguide conduit 362a. Accordingly, the electrodes 392a, 396a may provide a standoff distance with respect to a waveguide inserted in the waveguide conduit 362a. Further, the waveguide conduit 362a may include a distal stop, which may be similar to distal stops discussed herein with respect to one or more other embodiments.

In another embodiment, shown in FIG. 19B, a distal portion 370b may have side-by-side prong tips of electrodes 392b and 396b that are offset from each other horizontally in relation to waveguide conduit 362b and are separated by insulation tube 394b. Waveguide conduit 362b is shown with sight hole 399b. The electrodes 392b, 396b may provide a standoff distance, and the waveguide conduit 362b may include a distal stop, as discussed herein with respect to one or more other embodiments.

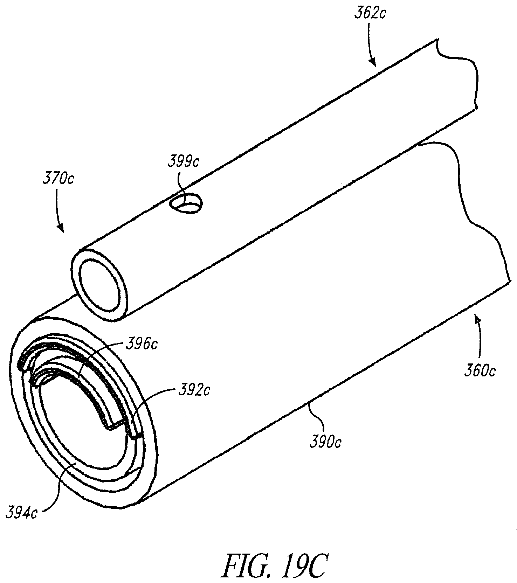

In another embodiment, shown in FIG. 19C, a distal portion 370c may have stacked concentric tips of electrodes 392c and 396c that are separated by insulation tube 394c. The tips of electrodes 392c, 396c are spaced apart from one another vertically by the insulation tube 394c. Waveguide conduit 362c is shown with sight hole 399c. The electrodes 392c, 396c may provide a standoff distance, and the waveguide conduit 362c may include a distal stop, as discussed herein with respect to one or more other embodiments.

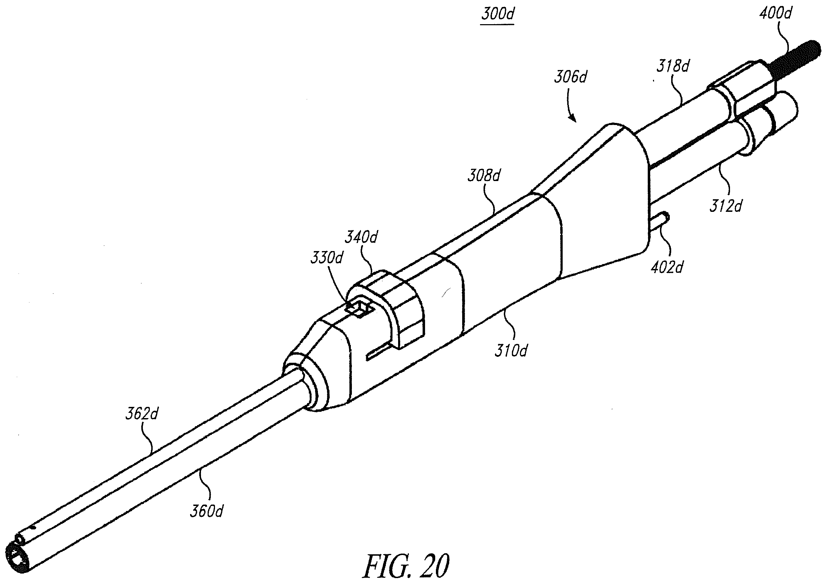

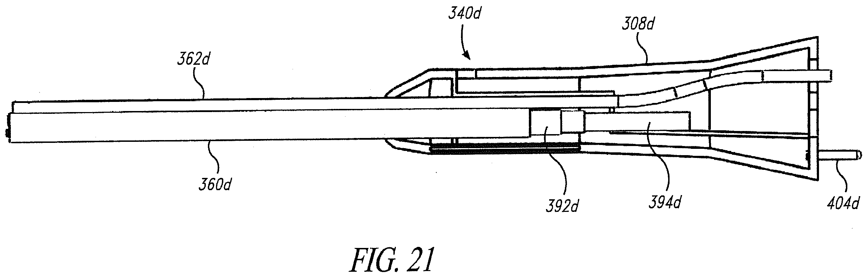

Still another handpiece 300d according to the present disclosure, shown in FIGS. 20-21, is similar to the handpiece 300 of FIGS. 12-18 but without push-button energy control. Instead, both laser energy and RF energy are controlled using one or more foot-switch controls that are connected to standard-sized electrode pin 402d (FIG. 20) and pin 404d (FIG. 21). One of pins 402d and 404d is connected to inner electrode 394d while the other of the pins 402d and 404d is connected to outer electrode 392d by one or more wires (not shown). A strain relief assembly (e.g., a flexible boot or strain relief boot, coil spring, tapered coil spring) 400d is shown attached to or otherwise communicatively coupled to the waveguide lock 318d in FIG. 20. A waveguide may passed through the strain relief assembly 400d and into the handpiece 300d (e.g., through the waveguide lock 318d), and the strain relief assembly 400d protects the waveguide from kinking, which could otherwise result in failure of the waveguide. Another difference from handpiece 300 is that suction port 330d is open when suction cover 340d is moved proximally as shown in FIG. 20, which places handpiece 300d in the "suction off" condition.

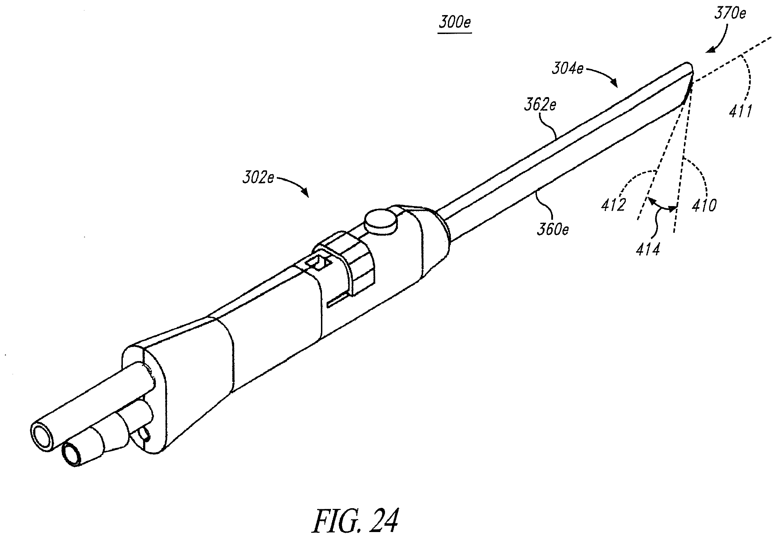

FIG. 24 is a schematic perspective view of still another modular handpiece 300e according to the present disclosure having a handle module 302e and a cannula module 304e with a combined suction and concentric electrode cannula 360e and a waveguide conduit 362e mounted on top of the cannula 360e. The distal end of the suction cannula 360e is beveled in this construction and has an angle 414 relative to vertical line 410 (which is perpendicular to the longitudinal axis 411 of the cannula 360e) and bevel line 412. Angle 414 typically ranges from zero to sixty degrees, preferably between ten to fifty degrees when a bevel is desired, and more preferably fifteen to forty five degrees to increase maneuverability, visibility, and ease of manipulation at a surgical site.

In one or more embodiments, one or more portions of the cannula module are shaped, dimensioned, positioned and arranged to perform additional functions, and may be formed as various different surgical tools, such as forceps, tweezers, scissors or graspers.

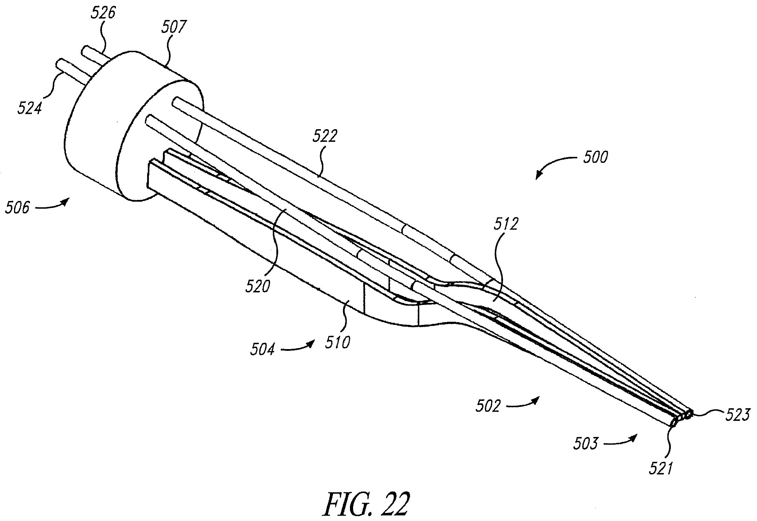

FIG. 22 is a schematic perspective view of yet another cannula module 500 according to the present disclosure that includes forceps arms 510 and 512, shown in a manually closed condition. Cannula module 500 is a bayonet-type configuration in this construction and includes an elongated distal portion 502 which terminates in a distal tip 503, a central portion 504, and a proximal portion 506. In one or more embodiments, electrodes 520 and 522 (which may be, for example, RF electrodes for electrosurgery) interconnect with separate proximal pins 524 and 526 within multi-lumen portion 507 and, in another embodiment, pins 524 and 526 represent the proximal portions of electrodes 520 and 522 which extend fully through electrode lumens in multi-lumen portion 507. Preferably, at least pins 524 and 526 are secured within portion 507 by adhesive, ultrasonic welding, or other fastening technique.

In some embodiments, electrodes 520 and 522 are hollow and terminate in distal openings 521 and 523. In one embodiment, a waveguide is passable through one of electrodes 520, 522 while the other of the electrodes 520, 522 defines a fluid passage usable for irrigation and/or suction (under positive or negative pressure, respectively). In yet other embodiments, one or both of hollow electrodes 620 and 622 are coaxial electrodes as described above in relation to FIGS. 12-21.

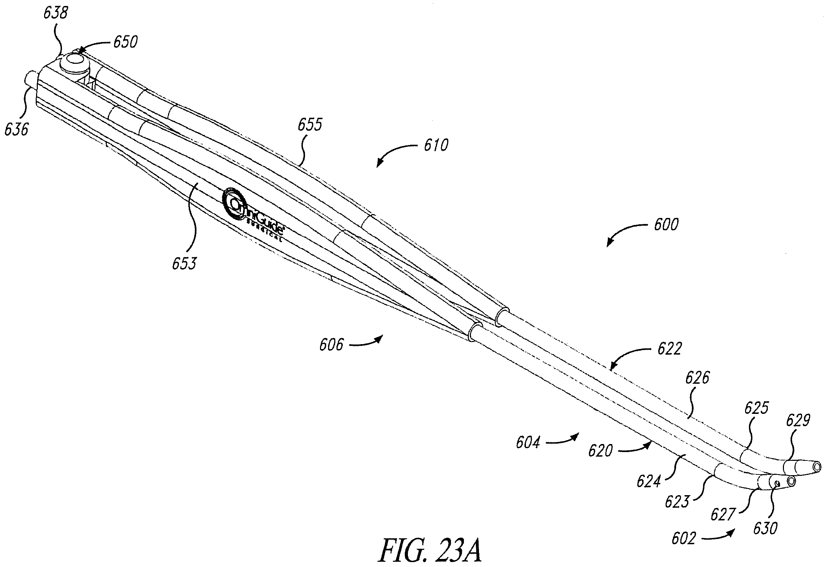

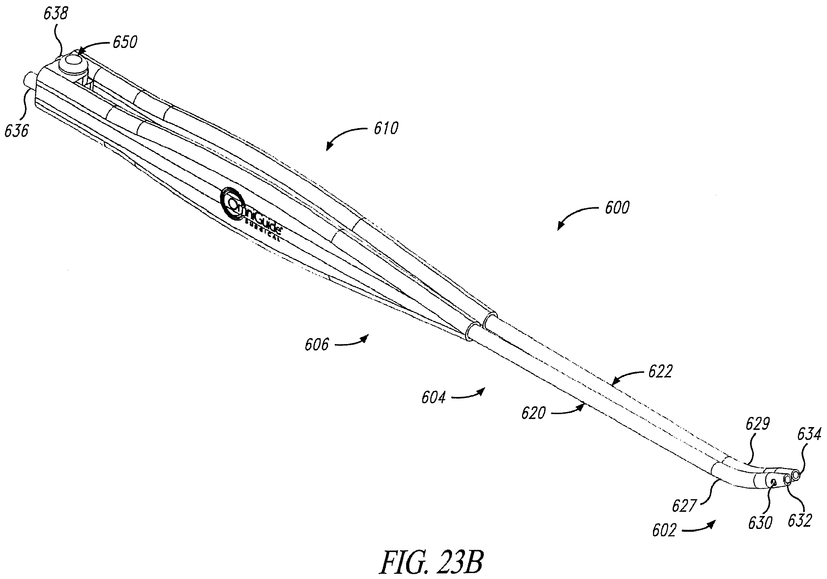

FIGS. 23A and 23B are schematic perspective views of yet another multifunction handpiece or device 600, which may be or include forceps, shown in the normally open (FIG. 23A) and manually closed (FIG. 23B) conditions. The forceps are integrated into a cannula module 608 and a handle module 610 in one stand-alone construction. Proximal electrode pins 636 and 638 plug into a standard RF connector in the stand-alone construction when RF surgical energy is desired.

In another embodiment, device 600 is a cannula module that is connectable to a separate handle module that is similar to one or more of those handle modules illustrated above in one or more of FIGS. 1-21.

As illustrated in FIGS. 23A-23B, multi-function device 600 includes cannula arms 620 and 622 having electrically insulative jackets 624 and 626 that extend distally to regions 623 and 625. In another embodiment, jackets 624 and 626 extend further into bend regions 627, 629, respectively. Proximal portion 606 fits within handle portion 610, which has graspable, and preferably electrically insulated, arm handle material 653 and 655 that are connected at joint 650. One or both of the arms 620, 655 are pivotable about joint 650 and, one or both of at least central cannula portion 604 and distal portion 602 are manually deflectable toward each other to achieve the closed condition shown in FIG. 23B. Although arms 620 and 622 are shown having substantially equal proportions, in other embodiments, one of the arms has a shorter length and/or smaller diameter than the other arm.

Arm 620 may include a waveguide passage that extends between a proximal tip and a distal tip of the arm 620, and that can accommodate a waveguide, as described herein with respect to one or more other embodiments. A waveguide viewing port 630 may be included in distal portion 602 (e.g., adjacent the distal tip of arm 620). A distal waveguide stop is provided in some embodiments and not in others, especially when it may be desirable to advance the waveguide beyond distal openings 632 and 634. One or more additional functions described in relation to any of FIGS. 1-21 can be added or omitted to the forceps embodiments 500 and 600 as desired. For example, arm 622 may include a suction passage that extends between a proximal tip and a distal tip of the arm 622, and that can be coupled (e.g., by a suction tube connector) to an external source of negative or suction pressure. Additionally, one or both of arms 620, 622 may include circular electrodes, which may be separated from one another by insulating tubes, as described herein with respect to one or more embodiments.

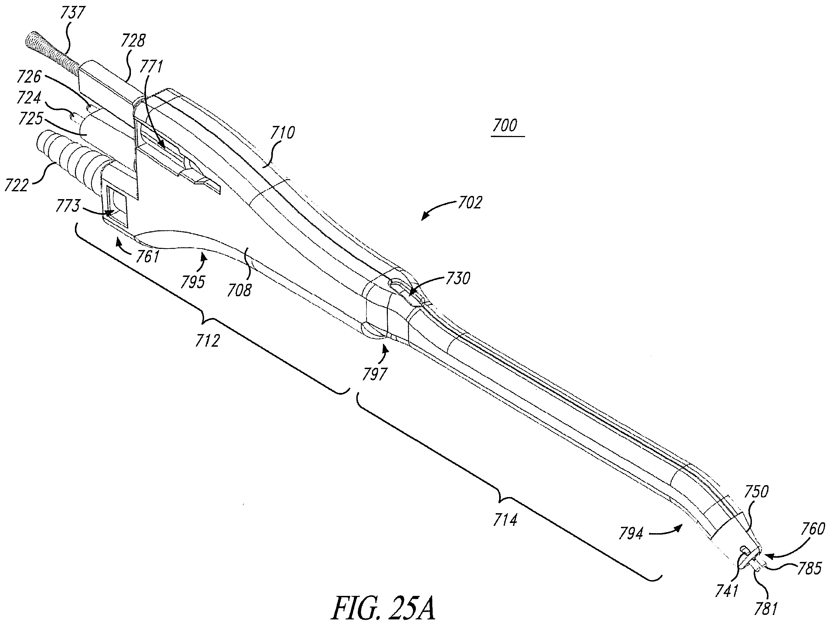

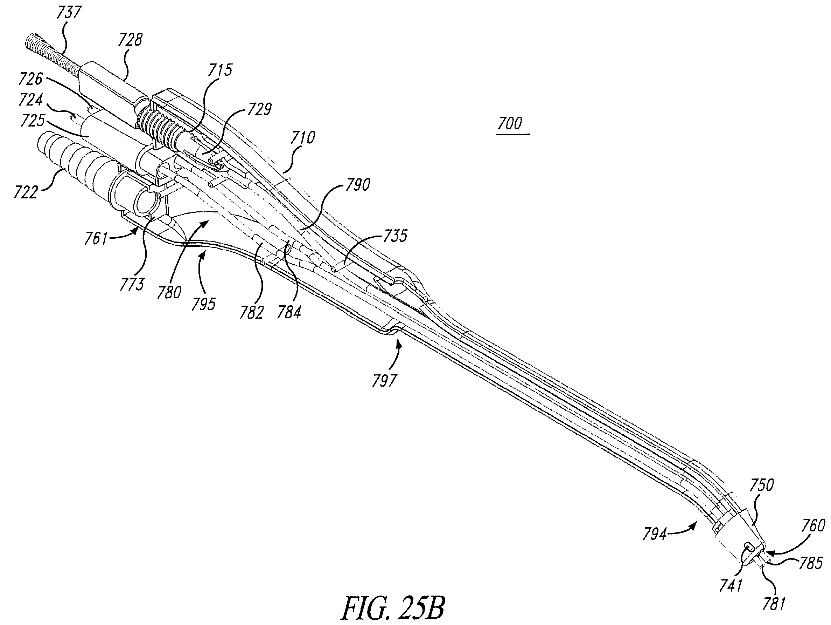

FIGS. 25A and 25B illustrate a multi-function surgical instrument (or "handpiece") 700, according to one or more embodiments of the present disclosure. In some respects, the handpiece 700 is similar to, and provides similar surgical functionalities as, one or more of the various handpieces described herein. There are, however, several notable differences, which will be described below. For example, instead of having separate a handle module and cannula module (e.g., handle module 12 and cannula module 14 of handpiece 10 shown in FIGS. 1-5B), the multi-function surgical instrument 700 includes a housing 702 which generally replaces the functions of, and alleviates the need for, separate handle and cannula modules.

The housing 702 may include a right-side section 708 and a left-side section 710, which together form a handle region 712, capable of being grasped by the hand of a user, and an elongate cannula region 714. Some or all of the cannula region 714 may be insertable into a patient's body, for example, through an oral cavity, through a nasal cavity, through an incision for laparoscopic surgical procedures, or the like. In that regard, the elongate cannula region 714 may have any length as may be desirable for use in various types of surgeries, including, for example, laparoscopic surgeries.

Each of the right-side section 708 and the left-side section 710 (which may also be referred to as "shells") may be formed, for example, by injection molding. The handpiece may then be assembled by placing the various components (which will be described in further detail below) within one or both of the right-side section 708 and the left-side section 710, and attaching the right-side section 708 and the left-side section 710 to one another. The walls of each of the right-side section 708 and left-side section 710 may have a substantially uniform thickness, which is desirable for injection molded components.

A plurality of housing connectors 735 may be formed on inner surfaces of the right-side and left-side sections 708, 710. The connectors 735 of the left-side section 710 may be attached to corresponding connectors on inner surfaces of the right-side section 708 to form a fluid-tight internal cavity 780 within the housing 702. The connectors 735 of the right-side and left-side sections 708, 710 may be, for example, snap-fit connectors which can provide a snap-fit attachment to one another by aligning the corresponding connectors and pressing the right-side and left-side sections 708, 710 toward one another, or the connectors 735 may be attached to one another with an adhesive such as an epoxy, or by any plastic welding technique, such as ultrasonic welding, RF welding, or the like, or by any other bonding technique.

A ribbed or barbed suction tubing connector 722 is provided at a proximal end of the handle region 712. The proximal ends of the right-side and left-side sections 708, 710 define openings to accommodate the suction tubing connector 722. A suction tubing connector receptacle 773 (an outer surface of which is shown in FIG. 25A, and inner surfaces of which are shown in FIG. 25B) is formed adjacent to the openings for the suction tubing connector 722, at proximal ends of the right-side and left-side sections 708, 710. When the right-side and left-side sections 708, 710 are joined to one another, with the suction tubing connector 722 positioned within the suction tubing connector receptacle 773, the suction tubing connector receptacle 773 forms a collar that securely holds the suction tubing connector 722.

The outer surface of the suction tubing connector receptacle 773 may be recessed with respect to the adjacent outer surfaces of the housing 702, as shown in FIG. 25A. By forming the suction tubing connector receptacle 773 with a recessed outer surface, it is possible to retain a substantially uniform wall thickness of the housing 702, which enables formation of the housing 702 by injection molding.

The suction tubing connector 722 may include a plurality of differently sized ribs or barbs, as shown, to accommodate, and to provide a fluid-tight seal when connected to, various sizes or diameters of suction hoses. The suction tubing connector 722 may be positioned at a lower portion of the handle region 712, as shown. For example, the suction tubing connector 722 may be attached to the handle region 712 such that the distal end of the suction tubing connector 722 is positioned within a lower extended portion (or "fluid collection portion") 761 of the housing 702, and of the interior fluid cavity 780. The lower extended portion 761 is formed at a bottom (or lower, when in use) portion of the handle region 712. During use, fluids or matter that are pulled into the interior fluid cavity 780, by suction, may flow toward the lower extended portion 761 where they are pulled into the suction tubing connector 722, e.g., when the user places a thumb or finger over a suction control port 730.

An electrode plug 725 is provided at the proximal end of the handle region 712. The electrode plug 725 may be, for example, a standard RF plug for bipolar electrosurgery applications, to which an external RF power source may be electrically coupled to provide electrical energy to proximal electrode pins 724, 726. The proximal ends of the right-side and left-side sections 708, 710 define openings to accommodate the electrode plug 725, and to securely hold the electrode plug 725 when the right-side and left-side sections 708, 710 are attached to one another. In an alternative embodiment, a cable may be hardwired directly to the electrodes 782, 784 within the housing 702, thus replacing one or more of the electrode plug 725 and the electrode pins 724, 726.

The multi-function surgical instrument 700 further includes a waveguide lock 728, which may be positioned above the electrode plug 725, which, in turn, is positioned above the suction tubing connector 722. The waveguide lock 728, electrode plug 725 and suction tubing connector 722 may be substantially vertically aligned at the distal end of the handle region 712, which may advantageously provide a balanced feel of the surgical instrument 700 in a user's hand.

A waveguide lock receptacle 771 (an outer surface of which is shown in FIG. 25A) is formed at proximal ends of the right-side and left-side sections 708, 710. When the right-side and left-side sections 708, 710 are joined to one another, the waveguide lock receptacle 771 forms an opening that receives the waveguide lock 728. As shown in the left-side section 710 of FIG. 25B, the waveguide lock receptacle 771 may include a threaded inner portion 715. The right-side section 708 includes a corresponding threaded portion (not shown) that, when aligned with and attached to the left-side section 710, forms a complete threaded portion into which the waveguide lock 728 may be threadably inserted. The waveguide lock 728 may include a collet 729. While the waveguide lock 728 is shown in FIG. 25A as including a collet 729, any locking mechanism suitable for locking a position of a waveguide, when inserted into the multi-function surgical instrument 700, may be utilized as the waveguide lock 728. Additionally, a flexible boot 737 may be included as part of, or otherwise attached to, the waveguide lock 728. The flexible boot 737 may be, for example, a flexible spring-like device through which a waveguide may be inserted, and which prevents the waveguide from being bent at a sharp angle, such as 90°, that may exceed a recommended bend tolerance of the waveguide.

The outer surface of the waveguide lock receptacle 771 may be recessed with respect to the adjacent outer surfaces of the housing 702, as shown in FIG. 25A, which enables formation of the housing 602 by injection molding, with a substantially uniform wall thickness. Some components of the multi-function surgical instrument 700, such as the waveguide lock 728, may be separately produced (e.g., by injection molding, or by any other suitable manufacturing technique) in a separate process than portions (e.g., the right-side and left-side sections or shells 708, 710) of the housing 702, which allows the portions of the housing 702 to have a relatively uniform thickness, and be sufficiently thin that the overall peripheral dimension e.g., diameter, of the neck or cannula region 714 is small (and suitable for use, for example, in oral cavity, laparoscopic, and other surgeries) while having interior space to accommodate the suction passage (which is defined by the inner walls of the housing 702), the waveguide conduit 790, and the electrical wiring or electrodes 782, 784. The uniform thickness advantageously allows injection molding to be used, and separating out certain components for separate production allows a thin wall size to be achieved, via the injection molding, resulting in a tip with a relative small lateral dimension while still tightly packing the suction passage, waveguide conduit 790 and electrodes 782, 784. Tight packing is further facilitated by the interior walls of the housing 702 forming the fluid cavity 780 rather than requiring inclusion of a separate tube to be housed in the body of the multi-function surgical instrument 700 (e.g., as done in earlier presented embodiments). In one or more embodiments, a waveguide conduit 790 is not included; instead, a waveguide may simply be routed through the housing 702, e.g., between the waveguide passage of the distal tip assembly 750 and an opening in the proximal end of the housing 702 (e.g., through the waveguide lock 728).

A waveguide may be passed through an interior lumen within the waveguide lock 728, which threadably engages the threaded portion 751 of the waveguide lock receptacle 771 in the handle region 712 of the housing 702. The collet 729 of the waveguide lock 728 presses on the waveguide, when inserted through the interior lumen of the waveguide lock 728, as the collet 729 advances distally, e.g., by threading the waveguide lock 728 into the threaded portion 715. As previously described herein, such advancement of the collet 729 may force outer surfaces of the collet 729 against a tapered interior surface, thereby forcing the collet 729 to press on the waveguide and hold the waveguide in a locked position. The waveguide lock 728 may be selectively releasable. For example, the waveguide may be released from the locked position by unthreading the waveguide lock 728, thereby retracting the waveguide lock 728 from the threaded waveguide lock receptacle 771, which allows the collet 729 to spread open as it is retracted from the tapered collar in the receptacle 771.

After components are placed into one or both of right-side and left-side sections 708, 710, then the matching edges of sections 708 and 710 are mated and secured together with an adhesive such as an epoxy, or by any plastic welding technique, such as ultrasonic welding, RF welding, or the like, or by any other bonding technique.

A suction control port 730 is established by corresponding openings in right-side and left-side sections 708, 710 after the right-side and left-side sections 708, 710 are secured together.

During use, a user typically grasps the exterior surface of the housing 702, at the handle region 712, with the palm and fingers of one hand, and then utilizes a thumb or a finger (e.g., the index finger) to control suction by covering suction control port 730 directly with the thumb. Using the thumb or finger, the user can thus selectively control suction through the interior fluid cavity 780. While a thumb or any finger may be used, control of suction may be most effectively and comfortably accomplished using the index finger.

By forming the housing 702 as two pieces, i.e. right-side section 708 and left-side section 710, the interior fluid cavity 780 may have a larger volume (e.g., as compared to a machined, monolithic handle unit) and may be formed by attaching the right-side and left-side sections 708, 710 to one another, the inner walls of which define boundaries of the interior fluid cavity 780. As such, a separate suction passage or suction conduit does not need to be provided through the housing 702, since the entire interior fluid cavity 780 may be utilized as a suction passage. Moreover, the larger volume of the interior fluid cavity 780 assists in, or otherwise facilitates, assembly of the multifunction surgical handpiece 700. For example, the larger volume can accommodate adjustable length electrodes (for example, the electrodes 782, 784 may include portions of coiled wire that is housed within the interior fluid cavity 780, and that can be uncoiled to provide an extended electrode length), and can further permit low machining tolerance parts (including, e.g., the electrodes) to be precisely positioned in a desired location within the handpiece 700.

FIG. 25B is a schematic perspective view of the multi-function surgical instrument 700, showing the left-side section 710 prior to attachment to the right-side section 708. The suction tubing connector 722, electrode plug 725, and waveguide lock 728 are shown inserted into the left-side section 710 during assembly of multi-function surgical instrument 700.

The multi-function surgical instrument 700 further includes a distal tip assembly 750 that may be attached to the distal end of the housing 702. For example, the distal tip assembly 750 may be inserted into corresponding openings formed at the distal ends of the right-side and left-side sections 708, 710, and securely attached to the housing 702 by attaching the right-side and left-side sections 708, 710 to one another. The distal tip assembly 750 may be substantially the same as, or similar to, the distal portion 70 of the handpiece 10 shown in FIG. 1. For example, the distal tip assembly 750 includes openings at the distal tip 760 that are communicatively coupled to respective waveguide, electrode, and suction passages, as described previously herein.

Two electrodes 782 and 784 may be routed through the distal tip assembly 750 (e.g., through corresponding electrode passages in the distal tip assembly 750, as described previously herein) and through the interior fluid cavity 780 that is formed by attaching the right-side and left-side sections 708, 710 to one another. The electrodes 782, 784 may be electrically coupled to the proximal electrode pins 724, 726, for example, within the electrode plug 725. For example, the distal ends of the proximal electrode pins 724, 726 may be hollow, and the proximal ends of the electrodes 782, 784 may be inserted into the proximal electrode pins 724, 726, thereby establishing electrical continuity. Alternatively, the electrodes 782, 784 may extend through the electrode plug 725, such that portions of the electrodes 782, 784 extend distally through the electrode plug 725, thereby serving as electrode pins capable of being electrically coupled to an external power supply. The electrode plug 725 may include recesses or counterbores for accommodating insulation termination (e.g., a point where insulation that surrounds the electrodes is stripped or otherwise terminated), while an exposed portion of the electrodes 782, 784 passes through an opening in the electrode plug 725. The recesses or counterbores further facilitate potting (e.g., with an adhesive) which prevents electrical arcs between the electrodes 782, 784 at or near the electrode plug 725.

Similarly, in one or more embodiments, the electrode passages in the distal tip assembly 750 may include a counterbore. The counterbore may provide a recess for accommodating insulation (e.g., insulation that surrounds the electrode) up to a termination point of the insulation (e.g., where the insulation is stripped from the electrode), while the stripped or exposed electrode may pass through a hole or passage in the counterbore. The recess provided by the counterbore further facilitates and accommodates potting (e.g., using an adhesive), which prevents electrical arcs between the electrodes 782, 784 near or through the distal tip assembly 750.

Portions of the electrodes 782, 784 (e.g., the portions within the housing 702) may be covered with electrically non-conducting insulation. The distal tips of electrodes 782, 784, which extend distally beyond the distal tip 660 of the distal tip assembly 750, are electrically exposed, i.e. they are not covered by insulation. While the distal tips of electrodes 782, 784 are shown as being substantially rounded, or circular in cross-section, the distal tips of electrodes 782, 784 are not limited to such a shape. The distal tips of electrodes 782, 784 may be any other shape and may be, for example, triangular in cross-section.

A suction passage is established between the suction tubing connector 722 and the suction opening at the distal tip 760 of the distal tip assembly 750 through the internal fluid cavity 780.

A waveguide conduit 790 is provided within, and extends through, the interior of the housing 702, between the waveguide lock 728 and the distal tip assembly 750. A waveguide may thus be inserted through the waveguide lock 728, where it will be received by the waveguide conduit 790. The waveguide may be advanced through the waveguide conduit 790 and into a waveguide passage in the distal tip assembly 750, until it reaches a desired position, at which point the waveguide lock 728 may be advanced (e.g., by threading the waveguide lock 728 into the threaded portion 751 of the waveguide lock receptacle 771) until the waveguide is locked in its desired position.

As described with respect to various embodiments herein, the distal tip assembly 750 may include a waveguide stop (e.g., waveguide stop 57, shown in FIG. 3). The waveguide stop may be formed by a tapered interior perimeter dimension inside the waveguide passage of the distal tip assembly 750, or by a stepped decrease from a wider inner perimeter dimension to a narrower inner perimeter dimension within the waveguide passage of the distal tip assembly 750, as previously described herein. Accordingly, the waveguide stop may prevent an inserted waveguide from advancing beyond the waveguide stop, which may be positioned proximal to the distal tip 760 of the distal tip assembly 750.

Similarly, as described herein with respect to one or more embodiments, the waveguide passage within the distal tip assembly 750 may have a flared shape near the distal tip 760, with an inner perimeter dimension that increases distally (e.g., from the waveguide stop to the waveguide opening at the distal tip 760 of the distal tip assembly 750), which accommodates a laser beam that has an increasingly larger diameter as the beam emerges from the distal tip of the waveguide.

Additionally, as described herein with respect to one or more embodiments, the distal tip 750 may include a sight hole 741. When a waveguide is inserted in the distal tip 750 and advanced distally to, or near, the waveguide stop within the distal tip 750, the waveguide may be viewable through the sight holes 741, which allows a user to visually confirm the positioning of the waveguide in the distal tip 750.

As described herein with respect to various embodiments, the distal tips of the electrodes 782, 784 may extend beyond the distal tip 760 of the distal tip assembly 750 to a distance up to about 5mm or more, and may preferably be within a range of 2mm, inclusive, to 5mm, inclusive. The distal ends of the electrodes 782, 784 thus define a standoff distance, which is the distance between the distal tip 760 of the distal tip assembly 750. The standoff distance may be selected to have any distance suitable to prevent tissue from being sucked into or from blocking the suction opening at the distal tip 760.

Further, as discussed previously herein with respect to various embodiments, the electrodes 782, 784, the waveguide opening and the suction opening may each have respective axes that are parallel with one another at the distal tip 760 of the distal tip assembly 750. As such, a laser delivery axis (e.g., the axis of the waveguide, when inserted, at the distal tip 760), an electrosurgery axis (e.g., the axes of electrodes 782, 784 extending from the distal tip 760), and a suction axis (e.g., the axis of the suction opening at the distal tip 760) are all parallel with one another, and spaced apart from one another. Accordingly, the laser energy delivered via the waveguide, and that is emitted from the waveguide opening, does not eclipse an electrosurgery area defined by the position of the distal tips of the electrodes 782, 784, and similarly does not eclipse a suction area at the suction opening of the distal tip 760.

The housing 702 may include one or more bends 794 along the cannula region 714, which may have a fixed bend angle as may be desirable in certain applications or types of surgeries.

The housing 702 may further include a distal handle bend 795 in the handle region 712 of the housing 702, which bends downward from a substantially flat portion of the handle region 712 to the lower extended portion 761 of the housing 702. The distal handle bend 795 may generally be an s-shaped curve (or "s-curve") having an inflection point at which the curved shape at the bottom of the housing 702 changes from being concave to convex. Additionally, a lower notch 797 may be formed by a bend that is generally positioned between the cannula region 714 and the handle region 712 of the housing 702. The lower notch 797 may be positioned opposite, or nearly opposite, to the suction control port 730. Multiple gripping positions are thus provided by the housing 702. For example, a user may grip the handle region 712, with the palm and fingers generally positioned between the distal handle bend 795 and the notch 797, when suction control is not needed. The user may, instead, grip the housing 702, with a thumb or a finger (e.g., the index finger) positioned at or near the suction control port 730, and with a finger (e.g., the middle finger) positioned in the notch 797, which provides suction control and may also provide a balanced feel, with the housing 702 substantially balanced about the finger in the notch 797. Additional curves or bends may be formed on an upper surface of the housing 702, as shown, and further enhance the ergonomic and balanced grip provided by the handle region 712 housing 702. For example, the upper surface of the housing 702 may generally include an s-curve, or a curve having an inflection point, that is positioned in the handle region 712 between the proximal end of the handle region 712 and the suction control port 730. The shape of the housing 702, including the distal handle bend 795 and the notch 797 thus defines an ergonomic handle design that can be comfortably gripped in multiple positions. Moreover, the vertical alignment of the suction tubing connector 722, the electrode plug 725 and the waveguide lock 728, facilitates balance of the multi-function surgical tool 700 when gripped about the distal handle bend 795 (e.g., in a first gripping position), and when gripped with one or more fingers positioned in the notch 797 (e.g., in a second gripping position). The ergonomic design and balanced feel of the multifunction surgical instrument 700 enables a user to comfortably hold and effectively use the instrument 700 for surgical procedures which may last for relatively long periods of time. The ergonomic design and balanced feel further aid in control of the instrument 700, by the user, throughout the duration of a surgical procedure.

One or more of the cannula modules of any of the various embodiments described above can also be adapted for manipulation by a robotic surgical system and/or surgical assist system. Examples of known robotic surgical systems utilizing lasers and other instruments are provided by Mohr in U.S. Patent Publication No. 2009/0171372, by Williams et al. in U.S. Patent Publication No. 2009/0248041 and by Prisco et al. in U.S. Patent Publication No. 2010/0249507, for example, all assigned to Intuitive Surgical Operations, Inc. and/or Intuitive Surgical, Inc. of Sunnyvale, California, which provides the Da Vinci™ robotic platform. Robotically assisted surgery through a single port utilizing an image capturing device and multiple surgical tools is described by Mohr in U.S. Patent No. 8,517,933.

System control settings for a surgical energy device typically include power settings such as at least one of power level, pulse rate, pulse width, pulse shape and duty cycle for delivery of electromagnetic radiation by the surgical energy device. In certain constructions, at least one fluid source delivers at least one gas or liquid to a target location.

In some constructions, the source of optical radiation is part of a laser surgery system such as the Intelliguide™ Laser System commercially available from OmniGuide, Inc. of Lexington, Massachusetts. The laser surgery system typically includes a human interface, including buttons, dials, knobs or a touch screen, which can be used to select the laser power settings in some constructions. Systems optionally includes at least one fluid source, which generates a first fluid flow and is part of the laser surgical system in some constructions. At least one fluid flow valve, actuated via a control such as a footswitch, and a chiller/heater are provided for the fluid source in some constructions. It may also optionally include a second fluid source with its own independent fluid flow valve and chiller/heater. In other constructions, knobs, buttons or touch screen control replaces one or more foot controls. When the energy source includes a laser and is part of a laser surgery system, the laser surgery system may also include a flexible waveguide for delivering laser radiation to the surgical site such as the BeamPath™ flexible fiber waveguides available from OmniGuide, Inc. The waveguide is hollow in some constructions. An articulated arm may also be used to deliver free space laser beams to the surgical site. One source of fluid may directed to the center of the hollow waveguide and a second source to an annular area surrounding the waveguide, such as described in more detail by Fuflyigin et al. in U.S. Published Application No. 2015/0202005A1.

Another approach is to make the energy spot size smaller by focusing optical energy using lenses or by controlling the distance between tissue and the energy exit point, such as when an optical fiber is used to guide energy to the tissue. Certain techniques of altering the energy spot size utilizing optical components in a handpiece are provided by Shurgalin et al. in U.S. Patent Publication No. 2013/0064515.

Yet another approach is to direct cooling fluid preferentially around the spot relative to its center, as further described by Fuflyigin et al. in U.S. Published Application No. 2015/0202005A1.

The relationship between tissue temperature and tissue change, for both tissue effect and visual effect, is shown in Table I:

**TABLE I.**

| TEMPERATURE | TISSUE EFFECT | VISUAL EFFECT |
|---|---|---|
| 37°C-60°C | Heating | No Change |
| 60°C-90°C | Denaturation/Onset of Coagulation | White/Grey |
| 90°C-100°C | Drying/Puckering | Wrinkling/Puckering |
| 100°+C | Vaporization (Cutting/Ablation) | Golden/Char/Smoke |

When water in tissue reaches 100°C, water vapor and solid particulates are created, which appears as "smoke." Whether cutting or ablation occurs depends on several system control settings as described in more detail below.

Laser surgery typically utilizes long, thin, flexible solid or hollow waveguides to deliver specific wavelengths of electromagnetic radiation. Solid core silica fibers, for example, are utilized to guide wavelength of KPT (532 nm), Nd:YAG (1.06 µm), Ho:YAG (2.1 µm) and Tm: YAG (2 µm) lasers for various medical applications. For CO₂ laser beams (approximately 10.6 µm wavelength), hollow waveguides are useful, as the CO₂ wavelength is generally highly absorbed in materials traditionally used for optical fibers, such as silicates and thermoplastic polymers. A high omnidirectional reflector is disclosed in U.S. Patent No. 6,130,780 to Joannopoulos et al.

Flexible hollow waveguides are manufactured in some techniques by drawing structured thermoplastic preforms. Examples of such a structure are described by Harrington et al. in U.S. Patent No. 5,440,664 and by Fink et al. in U.S. Patent Nos. 6,463,200 and 7,311,962, in which a dielectric stack of materials having different refractive indices is arranged in concentric cylinders about the waveguide axis thus providing the mirror structure that guides the radiation. Flexible hollow waveguides drawn from structured thermoplastic preforms are also disclosed in U.S. Patent Nos. 7,272,285 to Benoit et al. and 7,295,734 to Bayindir et al., as well as in the following U.S. Patents assigned to OmniGuide, Inc.: 6,788,864 by Ahmad et al.; 6,801,698 by King et al.; 6,898,359 by Soljacic et al.; and 7,142,756 by Anderson et al.

At times, certain surgical uses of energy delivery devices such as waveguides may result in tissue debris, fluid, or smoke being generated. Such tissue debris may absorb delivered energy, including backscattered laser energy, and heat or otherwise interfere with the waveguide. Such tissue debris may impede or slow normal passive cooling resulting from thermal dissipation, and/or impede more active cooling resulting from delivered fluid, including gas flow through the waveguide core. The combination of increased heating and reduced cooling may overheat and thus damage the waveguide.

One approach to protect the portion of the surgical energy device is to flow fluid through a conduit such as hollow core waveguides. Gas flow may be used for clearing tissue debris and blood during tissue cutting, for cooling the waveguide and for therapeutic reasons such as assisting tissue coagulation. The gas flowing out of the waveguide may also assist in keeping the waveguide core from clogging and from damage due to the splattering, splashing, or deposition of tissue debris, including smoke and fluids. Protection of the waveguide distal end may also be achieved by a tip attached to the waveguide distal end, such as disclosed by Temelkuran et al. in U.S. Patent Nos. 7,167,622 and 7,331,954, by Goell et al. in U.S. Patent No. 8,280,212, and by Anastassiou et al. in U.S. Patent Publication No. 2014/0088577, all assigned to OmniGuide, Inc. of Lexington, Massachusetts. A solid distal optical tip which may have one or more cutting edges is described in U.S. Patent No. 5,951,543 by Brauer.

FIG. 14A of PCT Publication No. WO2016/044640A1 by Graham et al., which corresponds to FIG. 8A of Published Application No. 2015/0202005A1 by Fuflyigin et al., illustrates a waveguide 1300 for optical radiation including an opto-mechanical system to control the spot size of energy applied to the tissue for a diverging energy beam 1302 coming out of the waveguide 1300. One example is a hollow core fiber currently available from OmniGuide, Inc. that has an inner diameter of approximately 320 microns. In one construction, the spot diameter at distal tip 1304 is 320 microns, the spot diameter 1306 at distance 1308 of 1mm is 400 microns, the spot diameter 1310 at distance 1312 of 2mm is 485 microns, the spot diameter 1314 at distance 1316 of 3mm is 570 microns, the spot diameter 1318 at distance 1320 of 2cm is 2.0mm, and the spot diameter 1322 at distance 1324 of 3cm is 2.8mm.

In one construction, a waveguide tip with a variable cantilevered distal end portion length allows a user to select a spot size, such as by using movable extension 265 in Figures 7A and 7B of Anastassiou et al. in U.S. Patent Publication No. 2014/0088577. In another construction, an optical component-type tip such as disclosed by Shurgalin et al. in U.S. Patent Publication No. 2013/0064515 is combined with the movable extension of Anastassiou et al. to adjust spot size and resulting thermal effects during a surgical procedure.

The illustration of the diverging cone 1302, FIG. 14A of PCT Publication No. WO2016/044640A1 by Graham et al., of radiation emerging from the waveguide 1300 illustrates how selecting the stand-off distance determines the spot size. The spot size may be determined approximately by directing the laser at a wooden tongue depressor and observing charring of the wood, for example. The spot size for a given waveguide beam divergence may be set during manufacturing, after the product has been sold but before surgery, by the surgical staff, or after or during a procedure. It may be possible to set the stand-off distance once, or many times. The stand-off distance may be set using a push or pull mechanism in the conduit.

The spot size of the laser radiation emitted from the distal tip affects the power density of the laser energy and thereby defines laser tissue interaction, such as cutting or ablation mode, as well as the rate of cutting or ablation. In general, a beam exiting an optical waveguide diverges as shown in FIG. 14A of PCT Publication No. WO2016/044640A1 by Graham et al. Therefore, spot size may be controlled by setting a distance between an exit point of the laser radiation and the tissue, i.e. by control of the stand-off distance.

Another way to control the distance between waveguide and the tissue may be by using a proximity sensor built into the waveguide, jacket, or conduit, or in the distal tip of the instrument. This proximity sensor may measure a distance to the tissue and provide a feedback to the user or computer interface. Distance may be controlled by the user or pre-programmed into a computer that automatically maintains a preset distance by adjusting the position of the manipulator.

FIGS. 15B and 15C of PCT Publication No. WO2016/044640A1 by Graham et al. are schematic illustrations of low power and higher power, respectively, with a CO₂ laser waveguide 1330 spaced relatively far from tissue. At low power input of 2-4 watts and a distance 1332, FIG. 15B, of 2-3cm from tissue TS, diverging CO₂ laser beam 1334 causes superficial ablation 1336 having a relatively shallow depth as represented by arrows 1338 and 1340. By comparison, at a higher power input of 15-20 watts and a distance 1342, FIG. 15C, of 3-5cm from tissue TS, diverging CO₂ laser beam 1344 causes ablation and coagulation 346 having a deeper depth as represented by arrow 1348.

FIGS. 15D and 15E of PCT Publication No. WO2016/044640A1 by Graham et al. are schematic illustrations of low power and higher power, respectively, with a CO₂ laser waveguide 1330 spaced relatively close to tissue TS at a distance of 0.2-0.3cm in both examples. At a first power input of 6-12 watts, diverging CO₂ laser beam 354, FIG. 15D, causes fine cutting 1356 having a relatively shallow depth as represented by arrows 1358 and 1360. By comparison, at a second, higher power input of 12-20 watts, diverging CO₂ laser beam 1364 causes cutting 1366 having a deeper depth as represented by arrow 1368.

While many of the above-described embodiments involve optical waveguides for laser surgery and/or RF electrodes, this is not a limitation of the disclosure. Modular handpieces according to the present disclosure are also suitable for use with other types of surgical energy such as electrosurgery utilizing microwave energy. FIGS. 16A and 16B of PCT Publication No. WO2016/044640A1 by Graham et al. are schematic side views of alternative single-use electrosurgical energy guides for use with a handpiece according to the present disclosure. Guide 2000, FIG. 16A, has an energizable distal tip 2002 electrically connected to a banana plug connector 2004 by an elongated insulated section 2006. Guide 2010, FIG. 16A of PCT Publication No. WO2016/044640A1 by Graham et al., has an energizable distal tip 2012 electrically connected to a two-prong plug connector 2014 by an elongated insulated section 2016. Distal tips 2002 and 2012 are insertable through an inner cannula of a handpiece according to PCT Publication No. WO2016/044640A1 by Graham et al. when the distal opening of the handpiece, such as opening 120 shown in FIGS. 7B and 7C, is enlarged to admit the energizable tips therethrough. In other constructions, the inner cannula serves as a working channel or lumen through which mini-laparoscopic instruments such as micro-debriders, cutting tools, forceps, biopsy or drug delivery needles, or other devices preferably having outer diameters less than 5mm can be utilized as desired.

Although specific features of the present disclosure are shown in some drawings and not in others, this is for convenience only, as each feature may be combined with any or all of the other features in accordance with the disclosure. While there have been shown, described, and pointed out fundamental novel features of the disclosure as applied to one or more preferred embodiments thereof, it will be understood that various omissions, substitutions, and changes in the form and details of the devices illustrated, and in their operation, may be made by those skilled in the art without departing from the spirit and scope of the disclosure. For example, it is expressly intended that all combinations of those elements and/or steps that perform substantially the same function, in substantially the same way, to achieve the same results be within the scope of the disclosure. Substitutions of elements from one described embodiment to another are also fully intended and contemplated. It is also to be understood that the drawings are not necessarily drawn to scale, but that they are merely conceptual in nature.

It is the intention, therefore, to be limited only as indicated by the scope of the claims appended hereto. Other embodiments will occur to those skilled in the art and are within the following claims.

The various embodiments described above can be combined to provide further embodiments. To the extent that they are not inconsistent with the specific teachings and definitions herein, all of the U.S. patents, U.S. patent application publications, U.S. patent applications, foreign patents, foreign patent applications and non-patent publications referred to in this specification and/or listed in the Application Data Sheet which are owned by OmniGuide, Inc., including but not limited to U.S. patent application Serial No. 62/337,291 filed May 16, 2016, are incorporated herein by reference, in their entirety. Aspects of the embodiments can be modified, if necessary, to employ systems, circuits and concepts of the various patents, applications and publications to provide yet further embodiments.

These and other changes can be made to the embodiments in light of the above-detailed description. In general, in the following claims, the terms used should not be construed to limit the claims to the specific embodiments disclosed in the specification and the claims, but should be construed to include all possible embodiments along with the full scope of equivalents to which such claims are entitled. Accordingly, the claims are not limited by the disclosure.

## Claims

1. A multi-function surgical instrument (700), comprising:
a housing (702) having a proximal end and a distal end, the housing including a handle portion (712) that forms the proximal end and a cannula portion (714) that forms the distal end, the housing delimiting an interior fluid cavity (780) and a suction control port (730) in fluid communication with the interior fluid cavity;
a distal tip assembly (750) physically coupled to the cannula portion (714) at the distal end of the housing (702), the distal tip assembly including:
a waveguide passage;
a suction passage; and
at least one electrode passage;
a waveguide conduit (790) that extends from the waveguide passage of the distal tip through the cannula portion (714) and into the handle portion (712); and
at least one electrode (782, 784) that extends from the at least one electrode passage of the distal tip through the cannula portion and into the handle portion.

2. The multi-function surgical instrument of claim 1 wherein the housing includes:
a waveguide lock receptacle (711) that extends into the handle portion from the proximal end of the housing, the waveguide lock receptacle is aligned with the waveguide conduit;
a suction tube connector receptacle that extends into the handle portion from the proximal end of the housing; and
an RF electrode plug opening at the proximal end of the housing.

3. The multi-function surgical instrument of claim 2 wherein the suction tube connector receptacle is positioned proximate to a bottom of the housing, and the RF electrode plug opening is positioned between the suction tube connector receptacle and the waveguide lock receptacle.

4. The multi-function surgical instrument of claim 3, further comprising:
a waveguide lock (728) that extends at least partially into the waveguide lock receptacle (711), the waveguide lock including a central lumen that is aligned with the waveguide conduit;
a suction tube connector that extends at least partially into the suction tube connector receptacle; and
an RF electrode plug (725) that extends at least partially into the RF electrode plug opening.

5. The multi-function surgical instrument of claim 4 wherein the waveguide lock (728), the suction tube connector, and the RF electrode plug (725) are aligned with one another along the proximal end of the handle portion, and the housing includes a distal handle bend (795) positioned along the bottom of the housing, the distal handle bend (795) forms at least a portion of an ergonomic gripping surface of the handle portion.

6. The multi-function surgical instrument of any of claims 4 or 5, further comprising:
a flexible boot (737) coupled to a proximal end of the waveguide lock, the flexible boot having a waveguide passage that is aligned with the central lumen of the waveguide lock (728), the flexible boot prevents a waveguide, if any, inserted into the waveguide lock through the flexible boot from being bent at a right angle.

7. The multi-function surgical instrument of any of claims 2 through 4 wherein the housing includes a fluid collection (761) portion adjacent to the proximal end and to the bottom of the housing (702), and the suction tube connector receptacle extends into the fluid collection portion.

8. The multi-function surgical instrument of any of claims 1 through 5 wherein each of the waveguide passage, the suction passage, and the at least one electrode passage are aligned along respective axes, each of which is spaced apart from, and parallel to, one another.

9. The multi-function surgical instrument of claim 8 wherein the at least one electrode (782, 784) extends distally to a distance between 2mm, inclusive, to 5mm, inclusive, beyond a distal tip (760) of the distal tip assembly (750).

10. The multi-function surgical instrument of any of claims 1 through 5 wherein the housing defines a notch (797) on a lower surface of the housing, between the handle portion (712) and the cannula portion (714).

11. The multi-function surgical instrument of any of claims 1 through 5 wherein the waveguide passage of the distal tip assembly includes a waveguide stop, the waveguide stop prevents advancement beyond the waveguide stop of a waveguide, if any, inserted into the waveguide passage.

12. The multi-function surgical instrument of claim 11 wherein the distal tip assembly (750) includes a waveguide sight hole (741) that enables viewing of the waveguide, if any, inserted into the waveguide passage at the waveguide stop.

13. A method of manufacturing a multi-function surgical instrument (300; 700), comprising:
forming a first housing side-section (308; 708) having a proximal end and a distal end, the first housing side-section including a first portion of a waveguide lock receptacle and a first portion of a suction tubing connector receptacle;
forming a second housing side-section (310; 710) having a proximal end and a distal end, the second housing side-section including a second portion of the waveguide lock receptacle and a second portion of the suction tubing connector receptacle;
positioning a distal tip assembly in an interior portion of at least one of the first housing side-section and the second housing side-section, the distal tip assembly including a waveguide passage, a suction passage, and at least one electrode passage;
positioning a waveguide conduit (362; 790) in the interior portion of the at least one of the first housing side-section and the second housing side-section, the waveguide conduit extending from the first portion of the waveguide lock receptacle to the distal end of the first housing side-section and into the waveguide passage of the distal tip assembly, or from the second portion of the waveguide lock receptacle to the distal end of the second housing side-section and into the waveguide passage of the distal tip assembly;
positioning at least one electrode in the interior portion of the at least one of the first housing side-section and the second housing side-section, the at least one electrode extending from the proximal end to the distal end of the at least one of the first housing side-section and the second housing side-section, and extending at least partially through the at least one electrode passage of the distal tip assembly; and
attaching the first housing side-section (308; 708) to the second housing side-section (310; 710).

14. The method of claim 13 wherein attaching the first housing side-section (308; 708) to the second housing side-section (310; 710) includes attaching the first housing side-section to the second housing side-section by RF welding.

15. The method of claim 14 wherein forming the first housing side-section and forming the second housing side-section comprises:
forming the first housing side-section (308; 708) by injection molding; and
forming the second housing side-section (310; 710) by injection molding.

16. The method of claim 13, further comprising:
positioning a suction tube connector (312; 722) between the first portion of the suction tubing connector receptacle and the second portion of the suction tubing connector receptacle, prior to the attaching the first housing side-section to the second housing side-section.
